# EUROPÄISCHE PATENTANMELDUNG

(11) **EP 2 286 908 A2**
(43) Veröffentlichungstag der Anmeldung: **23.02.2011**
(21) Anmeldenummer: 10191925.6
(22) Anmeldetag: 19.11.2010
(51) Int. Cl.: B01F 17/28, A61K 8/34, A61K 8/44, A61Q 19/00, B01F 17/38

(54) **Lösungsvermittler für kosmetische Zubereitungen**

(71) Anmelder: Symrise AG, 37603 Holzminden (DE)
(72) Erfinder: Issleib, Martina, 22955, Hoisdorf (DE); Claus, Jürgen, 37639, Bevern (DE); König, Martina, 21029, Hamburg (DE); Johncock, William, 21465, Reinbek (DE); Ohrmann, Rolf, 21255, Tostedt (DE)
(74) Vertreter: Eisenführ, Speiser & Partner

(57) **Zusammenfassung**

Die vorliegende Erfindung betrifft eine Mischung umfassend (i) ein oder mehrere 1,2-Alkandiole mit 5 bis 8 C-Atomen und (ii) ein oder mehrere Natrium- oder Kalium-R^{alk}-sarcosinate, wobei das Strukturelement R^{alk} Alkanoyl, Alkenoyl oder Alkadienoyl mit 12 bis 22 C-Atomen bedeutet, sowie gegebenenfalls (iii) ein oder mehrere Natrium- oder Kalium-C10-C16-Alkyl-sulfoacetate. Die Erfindung betrifft ferner bestimmte Zusammensetzungen und kosmetische Zubereitungen, insbesondere Körperpflegemittel, enthaltend eine solche Mischung oder Zusammensetzung sowie Verfahren zu deren Herstellung. Ferner betrifft die vorliegende Erfindung die Verwendung einer solchen Mischung als Lösungsvermittler und zur Erhöhung der Transparenz bzw. zur Verringerung der Trübung einer Zubereitung enthaltend eine oder mehrere weitere organische Substanzen.

## Beschreibung

Die vorliegende Erfindung betrifft eine Mischung umfassend (i) ein oder mehrere 1,2-Alkandiole mit 5 bis 8 C-Atomen und (ii) ein oder mehrere Natrium- oder Kalium-R^{alk}-sarcosinate, wobei das Strukturelement R^{alk} Alkanoyl, Alkenoyl oder Alkadienoyl mit 12 bis 22 C-Atomen bedeutet, sowie gegebenenfalls (iii) ein oder mehrere Natrium- oder Kalium-C10-C16-Alkyl-sulfoacetate. Die Erfindung betrifft ferner bestimmte Zusammensetzungen und kosmetische Zubereitungen, insbesondere Körperpflegemittel, enthaltend eine solche Mischung oder Zusammensetzung sowie Verfahren zu deren Herstellung. Ferner betrifft die vorliegende Erfindung die Verwendung einer solchen Mischung als Lösungsvermittler und zur Erhöhung der Transparenz bzw. zur Verringerung der Trübung einer Zubereitung enthaltend eine oder mehrere weitere organische Substanzen.

Kosmetische Zubereitungen bzw. Körperpflegemittel sind regelmäßig trüb, opal oder transparent und liegen häufig in Form von Emulsionen vor.

Lipophile Stoffe, wie beispielsweise Vitamine, Parfümöle oder UV-Lichtschutzfilter, lassen sich vielfach nur schwer in kosmetische Zubereitungen einarbeiten, insbesondere dann, wenn diese einen überwiegend polaren Charakter aufweisen, beispielsweise auf Grund eines entsprechend hohen Anteils an Wasser, bzw. an Wasser in Kombination mit Ethanol, Glycerin und/oder C1-C4-Alkandiolen. In solchen Fällen kommen regelmäßig Lösungsvermittler zum Einsatz, bei denen es sich um einzelne Stoffe oder Mischungen mit mittleren HLB-Werten handelt, die also gewissermaßen eine Brücke von der polaren Umgebung zum unpolaren Substrat bilden. Sehr effektive Hydrotrope stellen die Sulfonate kurzkettiger Alkylaromaten, wie z.B. Toluol- oder Cumolsulfonat dar, die wegen ihrer unzureichenden hautkosmetischen Verträglichkeit aber im Bereich der Kosmetik keine Bedeutung haben. Andere kosmetische Solubilisatoren, wie z.B. spezielle hydrophilisierte Öle, sind zwar hautverträglich, besitzen aber kein ausreichendes Lösungsvermögen und/oder ein schlechtes Kälteverhalten, d.h. zeigen schon bei Raumtemperatur die Tendenz zur Austrübung. Aus diesem Grunde besteht vor allem in der kosmetischen Industrie der Wunsch nach neuen Lösungsvermittlern, die frei von den oben geschilderten Nachteilen sind.

Emulsionen werden üblicherweise aus zwei flüssigen Phasen geformt, die nicht mischbar sind. Bei der Herstellung einer Emulsion wird eine Phase in fein verteilter Form in der anderen Phase dispergiert. Man unterscheidet hauptsächlich zwei Typen von Emulsionen, nämlich "Wasser-in-Öl-" und "Öl-in-Wasser-"Emulsionen. Bei der Öl-in-Wasser-Emulsion (O/W-Emulsion) stellt das Öl die innere Phase dar, die in der äußeren (Wasser-)Phase dispergiert ist. Die Eigenschaften der entsprechenden Emulsion werden im Wesentlichen durch die äußere Phase bestimmt, so dass sich Wasser-in-Öl-Emulsionen eher wie Öle und 01-in-Wasser-Emulsionen eher wie wässrige Lösungen verhalten.

Im Stand der Technik sind bereits zahlreiche Emulgatoren, Solubilisatoren und Lösungsvermittler beschrieben.

WO 2004/075868 und WO 2007/147904 beschreiben O/W-Emulgatoren auf Basis von Kalium-Cetylphosphat, gehärteten Palmölglyceriden und Cetylalkohol.

DE 1 132 925 schlägt als Lösungsvermittler für in Wasser schwer lösliche Verbindungen bestimmte Salze der Cyclohexylsalicylsäure bzw. der Hippursäure vor.

DE 1 198 476 betrifft Lösungsvermittler für etherische Öle, um diese in Wasser löslich zu machen. Die dort vorgeschlagenen Lösungsvermittler umfassend neben einem alkoxylierten Teilester von Glycerin einen alkoxylierten Ester aus einem Dialkanolamin mit einer höheren Fettsäure.

DE 3432219 A1 beschreibt Lösungsvermittler für Parfümöle, die Anlagerungsprodukte sind von 10 bis 60 Mol Ethylenoxid an Umsetzungsprodukte von epoxidierten Triglyceridölen mit ein-oder mehrwertigen Alkoholen mit 1 - 6 C-Atomen. Gemäß DE 3432219 A1 sollen Parfümöle, die regelmäßig öllösliche etherische Öle sind oder enthalten, in wässrigen kosmetischen Zubereitungen solubilisiert werden, so dass parfümölhaltige klare Zubereitungen erhalten werden.

WO 01/90245 A1 offenbart Lösungsvermittler für lipophile Stoffe enthaltend (a) Anlagerungsprodukte von Ethylenoxid an Fettalkohole, (b) Anlagerungsprodukte von Ethylenoxid und Propylenoxid an Fettalkohole, und (c) Anlagerungsprodukte von Ethylenoxid an Triglyceride. Die in WO 01/90245 A1 beschriebenen Lösungsvermittler sollen insbesondere gegenüber Stoffen, wie z.B. Parfümölen, Vitaminen, UV-Lichtschutzfiltern und dergleichen ein verbessertes Lösungsvermögen aufweisen, bei Raumtemperatur flüssig und klar sein und einen Kältetrübungspunkt unterhalb von 10°C aufweisen

EP 1 588 693 A1 betrifft bestimmte Lösungsvermittler für organische UV-Filter, wobei die Lösungsvermittler als wesentliches Strukturelement ein oder mehrere aromatische Ringe aufweisen.

EP 2 008 708 A2 beschreibt Lösungsvermittler mit klarem Aussehen und sehr gutem Lösungsvermögen von lipophilen Stoffen wie Parfümölen, Vitaminen oder UV-Lichtschutzfiltern. Ein Lösungsvermittler gemäß EP 2 008 708 A2 ist bei Raumtemperatur flüssig und klar und enthält a) ein oder mehrere ethoxylierte Fettalkohole, b) ein oder mehrere ethoxylierte Triglyceride mit bestimmten Acylresten sowie c) Wasser.

JP 2004-250332 A offenbart Shampoo-Zubereitungen enthaltend eine Kombination von Pentan-1,2-diol und/oder Hexan-1,2-diol sowie ein oder mehrere Tenside, wobei als Tensid Natrium-Laurylsulfoacetat genannt ist.

DE 10 2008 031 205 A1 betrifft die Verwendung von natürlichen 1,3-Diolen in Haarbehandlungsmitteln. Die dort beschriebenen Haarbehandlungsmittel können die unterschiedlichsten Tenside aus diversen Tensidklassen enthalten, wobei unter den zahlreichen genannten anionischen Tensiden unter anderem C8-C24-Acylsarcoside und Alkylsulfoacetate mit Alkylresten umfassend 6 bis 22 C-Atome aufgeführt sind.

Häufig werden auch Polyethylenglycole als Lösungsvermittler eingesetzt. Diese können jedoch Hautirritationen hervorrufen und die Schutzfilm der Haut angreifen, so dass der Einsatz von Polyethylenglycolen in kosmetischen Zubereitungen, insbesondere für Menschen mit empfindlicher Haut, problematisch ist.

Es war die Aufgabe der vorliegenden Erfindung, eine Komponente (Einzelsubstanz oder Substanzmischung) anzugeben, die eine verbesserte Löslichkeit von wenig bzw. schlecht wasserlöslichen organischen Verbindungen in Wasser ermöglicht, d.h. die Löslichkeit solcher organischer Verbindungen erhöht.

Die gesuchte Komponente soll bei 25°C und 1013 mbar flüssig und klar sein. Ferner sollte die Komponente möglichst wenig gefärbt sein, vorzugsweise hellgelb oder idealerweise farblos sein.

Vorzugsweise sollte die Komponente die Herstellung von möglichst transparenten, d.h. klaren, Zubereitungen erlauben, vorzugsweise von transparenten, wässrigen kosmetischen Zubereitungen, die darüberhinaus eine gute bzw. verbesserte Lagerstabilität der entsprechenden kosmetischen Zubereitungen bewirken, insbesondere hinsichtlich deren Langzeitstabilität.

Daneben sollte die Komponente möglichst keinen unangenehmen Geruch aufweisen bzw. kosmetischen Zubereitungen keinen unangenehmen Geruch vermitteln, vorzugsweise sollte die Komponente höchstens einen geringen Eigengeruch aufweisen.

Die gesuchte Komponente sollte vorzugsweise bereits in geringer Einsatzdosierung effektiv sein und vorzugsweise universell zur Herstellung von kosmetischen Zubereitungen unterschiedlicher Viskosität geeignet sein und vorzugsweise eine sehr gute Hautverträglichkeit aufweisen.

Die vorliegende Erfindung betrifft entsprechend einen Lösungsvermittler, der hervorragend zur Herstellung von kosmetischen Zubereitungen mit hoher Lagerstabilität geeignet ist und der zudem in einer bevorzugten Ausgestaltung frei von Polyethylenglycol (PEG) ist und unter anderem auch deshalb sehr gut hautverträglich ist.

Gemäß einem ersten Aspekt der vorliegenden Erfindung wird die gestellte Aufgabe gelöst durch eine Mischung umfassend
(i) ein oder mehrere 1,2-Alkandiole mit 5 bis 8 C-Atomen,
(ii) ein oder mehrere Natrium- oder Kalium-R^{alk}-sarcosinate, wobei das Strukturelement R^{alk} Alkanoyl, Alkenoyl oder Alkadienoyl mit 12 bis 22 C-Atomen, vorzugsweise mit 16 bis 22 C-Atomen, bedeutet, und
(iii) optional ein oder mehrere Natrium- oder Kalium-C10-C16-Alkyl-sulfoacetate.

Die Verbindungen der Bestandteile (i), (ii) und (iii) einer erfindungsgemäßen Mischung sind an sich bekannte Substanzen, die jeweils auch kommerziell verfügbar sind.

Eine erfindungsgemäße Mischung enthält als Bestandteil (i) ein oder mehrere 1,2-Alkandiole mit 5 bis 8 C-Atomen. Vorzugsweise sind die 1,2-Alkandiole mit 5 bis 8 C-Atomen unverzweigte, d.h. geradkettige, Alkandiole, d.h. 1,2-n-Alkandiole. Bevorzugt sind dabei 1,2-Pentandiol (Pentan-1,2-diol), 1,2-Hexandiol und/oder 1,2-Octandiol. Weiter bevorzugt sind 1,2-Pentandiol und/oder 1,2-Hexandiol, besonders bevorzugt ist 1,2-Pentandiol, da insbesondere mit 1,2-Pentandiol (CAS-Nummer 5343-92-0; INCI: Pentylene Glycol) die besten Ergebnisse im Sinne der vorliegenden Erfindung erzielt wurden.

Eine erfindungsgemäße Mischung enthält als Bestandteil (ii) ein oder mehrere Natrium- oder Kalium-R^{alk}-sarcosinate, wobei das Strukturelement R^{alk} Alkanoyl, Alkenoyl oder Alkadienoyl mit 12 bis 22 C-Atomen bedeutet. Bei den Verbindungen des Bestandteils (ii) handelt es sich um die Na- oder K-Salze von N-Alkanoyl-N-methylglycin, N-Alkenoyl-N-methylglycin oder N-Alkadienoyl-N-methylglycin, entsprechend der IUPAC-Nomenklatur Natrium- oder Kalium-2-[Alkanoyl(methyl)amino]acetate, Natrium- oder Kalium-2-[Alkenoyl(methyl)amino]acetate bzw. Natrium- oder Kalium-2-[Alkadienoyl(methyl)amino]acetate.

Die Verbindungen des Bestandteils (ii) können als Umsetzungsprodukte von Sarcosin (N-Methylglycin) mit Alkancarbonsäuren, Alkencarbonsäuren oder Alkadiencarbonsäuren mit 12 bis 22 C-Atomen aufgefasst und durch folgende Strukturformel beschrieben werden: 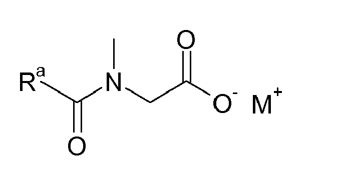 wobei R^{a} ein Alkylrest, ein Alkenylrest oder ein Alkadienylrest mit 11 bis 21 C-Atomen ist und M⁺ Natrium (Na⁺-Ion) oder Kalium (K⁺-Ion) bedeutet. R^{a} kann folglich keine, eine oder zwei C-C-Doppelbindungen aufweisen.

Als Beispiele für Verbindungen des Bestandteils (ii) seien exemplarisch Natrium-Lauroylsarcosinat (CAS-Nummer 137-16-6), Natrium-Myristoylsarcosinat, Kalium-Palmitoylsarcosinat, Natrium-Stearoylsarcosinat, Natrium-Oleoylsarcosinat, Kalium-Oleoylsarcosinat und Natrium-Linoleoylsarcosinat genannt.

Bevorzugt sind solche erfindungsgemäß eingesetzten Natrium- oder Kalium-R^{alk} -sarcosinate, in denen das Strukturelement R^{alk} Alkanoyl, Alkenoyl oder Alkadienoyl mit 16 bis 22 C-Atomen, weiter bevorzugt mit 16 bis 20 C-Atomen bedeutet, insbesondere bevorzugt mit 18 C-Atomen (entsprechend bedeutet R^{a} in der oben dargestellten Strukturformel bevorzugt einen Alkyl-, Alkenyl- oder Alkadienylrest mit 15 bis 19 C-Atomen, insbesondere bevorzugt mit 17 C-Atomen). Ebenfalls bevorzugt sind Natrium- oder Kalium-R^{alk}-sarcosinate, in denen das Strukturelement R^{alk} Alkenoyl bedeutet, dabei vorzugsweise n-Alkenoyl (entsprechend R^{a} = n-Alkenyl in der obigen Strukturformel). Weiter bevorzugt ist Natrium-Oleoylsarcosinat (CAS-Nummer 14351-62-3; INCI: Sodium Oleoyl Sarcosinate), da insbesondere mit Natrium-Oleoylsarcosinat hervorragende Ergebnisse im Sinne der vorliegenden Erfindung erzielt wurden.

Eine erfindungsgemäße Mischung enthält als optionalen Bestandteil (iii) ein oder mehrere Natrium- oder Kalium-C10-C16-Alkyl-sulfoacetate, d.h. Natrium- oder Kalium-Alkyl-sulfoacetate, in denen die Alkylgruppe 10 bis 16 C-Atome aufweist.

Die Verbindungen des Bestandteils (iii) können durch folgende Strukturformel beschrieben werden: 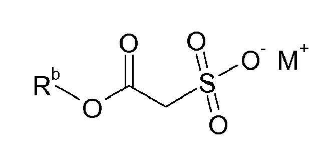 wobei R^{b} ein Alkylrest mit 10 bis 16 C-Atomen ist und M⁺ Natrium (Na⁺-Ion) oder Kalium (K⁺-Ion) bedeutet.

Die Verbindungen des Bestandteils (iii) können als Natrium- oder Kaliumsalze der Veresterungsprodukte von Sulfoessigsäure mit 1-Alkanolen mit 10 bis 16 C-Atomen aufgefasst werden.

Bevorzugt sind solche erfindungsgemäß eingesetzten Natrium- oder Kalium-C10-C16-Alkyl-sulfoacetate, in denen die Alkylgruppe unverzweigt ist (entsprechend R^{b} = n-Alkyl in der obigen Strukturformel). Weiter bevorzugt sind Natrium- oder Kalium-C10-C14-n-Alkyl-sulfoacetate, dabei insbesondere Natrium- oder Kalium-Laurylsulfoacetat und Natrium- oder Kalium-Myristylsulfoacetat. Besonders bevorzugt ist Natrium-Laurylsulfoacetat (CAS-Nummer 1847-58-1; INCI: Sodium Lauryl Sulfoacetate), da insbesondere bei Verwendung von Natrium-Laurylsulfoacetat zusammen mit den Bestandteilen (i) und (ii) weiter verbesserte Ergebnisse im Sinne der vorliegenden Erfindung erzielt wurden.

Bevorzugte erfindungsgemäße Mischungen sind solche, die als bevorzugte bzw. besonders bevorzugt gekennzeichnete Bestandteile umfassen, vorzugsweise in einer der vorstehend oder nachfolgend als bevorzugt bzw. besonders bevorzugt gekennzeichneten Ausgestaltungen, da mit diesen besonders gute Ergebnisse im Sinne der vorliegenden Erfindung erzielt wurden. Erfindungsgemäß besonders bevorzugt ist eine Mischung umfassend (i) 1,2-Pentandiol und (ii) Natrium-Oleoylsarcosinat sowie gegebenenfalls (iii) Natrium-Laurylsulfoacetat.

Es wurde überraschenderweise gefunden, dass (a) ein oder mehrere 1,2-Alkandiole mit 5 bis 8 C-Atomen in Kombination mit (b) einer oder mehreren Verbindungen aus der Gruppe bestehend aus Natrium- oder Kalium-R^{alk}-sarcosinaten, wobei das Strukturelement R^{alk} Alkanoyl, Alkenoyl oder Alkadienoyl mit 12 bis 22 C-Atomen bedeutet, vorzugsweise mit 16 bis 22 C-Atomen, Natrium- oder Kalium-C10-C16-Alkyl-sulfoacetaten und deren Mischung (d.h. ein Gemisch von (a) und (b)) verwendet werden kann
- als Lösungsvermittler für eine oder mehrere organische Substanzen,
- zur Erhöhung der Löslichkeit einer oder mehrerer organischer Substanzen,
- zur Verringerung der Trübung einer Zubereitung, vorzugsweise einer Dispersion und/oder Emulsion, enthaltend eine oder mehrere organische Substanzen,
und/oder
- zur Erhöhung der Transparenz einer Zubereitung, vorzugsweise einer Dispersion und/oder Emulsion, enthaltend eine oder mehrere organische Substanzen,
wobei die organischen Substanzen jeweils vorzugsweise einen log K_{OW} - Wert im Bereich von 1 bis 12 aufweisen, bevorzugt im Bereich von 2 bis 10, besonders bevorzugt im Bereich von 3 bis 8.

Es versteht sich, dass es sich bei diesen organischen Substanzen um weitere organische Substanzen handelt, die nicht den jeweiligen Definitionen der Bestandteile (i), (ii) oder (iii) einer erfindungsgemäßen Mischung genügen. Diese organischen Substanzen sind folglich keinem der Bestandteile (i), (ii) oder (iii) einer erfindungsgemäßen Mischung zuzurechnen.

Eine erfindungsgemäß bevorzugte Verwendung ist dadurch gekennzeichnet, dass eine oder mehrere organische Substanzen einen log K_{OW} - Wert im Bereich von 1 bis 12 aufweisen, vorzugsweise im Bereich von 2 bis 10, besonders bevorzugt im Bereich von 3 bis 8, und ausgewählt ist bzw. sind aus der Gruppe der zum Einsatz in kosmetischen Zubereitungen, vorzugsweise in Körperpflegemitteln, geeigneten Substanzen.

Bevorzugte Substanzgruppen, die entsprechende log K_{OW} - Werte aufweisen und zum Einsatz in kosmetischen Zubereitungen, insbesondere in Körperpflegemitteln, geeignet sind, sind Parfümöle, Riechstoffe, etherische Öle, Wirkstoffe, Antioxidantien, Kühlwirkstoffe, Deodorantwirkstoffe, UV-Filter, Vitamine, hautaufhellende Wirkstoffe und antiirritierende Wirkstoffe.

Der log-K_{OW} -Wert bezeichnet den Octanol-Wasser-Verteilungskoeffizienten. Im Rahmen des vorliegenden Textes bezeichnet der Begriff "log-K_{OW} -Wert" den anhand der jeweiligen molekularen Struktur berechneten Wert bei Verwendung des Softwareprogrammes "EPI Suite^{™} v4.00" von der United States Environmental Protection Agency, Washington DC, USA, welches wiederum das Softwareprogramm "KOWWIN™" nutzt, das nach der Atom/Fragment-Beitragsmethode arbeitet.

Die erfindungsgemäßen Mischungen sind bei 25°C und 1013 mbar flüssig und klar und zeichnen sich vorzugsweise, insbesondere in den als bevorzugt gekennzeichneten Ausgestaltungen, durch Kälte-Trübungspunkte unterhalb von +10°C aus.

Erfindungsgemäße Mischungen sind transparent und weisen insbesondere eine Trübung von weniger als 3 NTU (nephelometric turbidity units) auf gemäß DIN ISO EN 27027.

Ferner sind die erfindungsgemäßen Mischungen lediglich schwach gefärbt, im Regelfall transparent hellgelb. Die erfindungsgemäßen Mischungen weisen daneben keinen unangenehmen Geruch auf und zeigen lediglich einen schwachen Eigengeruch.

Die erfindungsgemäßen Mischungen weisen hinsichtlich Farbe und Geruch verbesserte Eigenschaften auf im Vergleich zu bislang üblichen Lösungsvermittlern.

In einer erfindungsgemäßen Mischung liegt das Gewichtsverhältnis von Bestandteil (i), dabei vorzugsweise 1,2-Pentandiol, zu Bestandteil (ii), dabei vorzugsweise Natrium-Oleoylsarcosinat, im Bereich von 5 : 1 bis 1 : 5, vorzugsweise im Bereich von 3 : 1 bis 1 : 3, bevorzugt im Bereich von 2 : 1 bis 1 : 2, weiter bevorzugt im Bereich von 3 : 2 bis 2 : 3, besonders bevorzugt im Bereich von 4 : 3 bis 3 : 4, ganz besonders bevorzugt im Bereich von 8 : 7 bis 7 : 8.

Eine erfindungsgemäße Mischung lässt sich bereits bei geringer Einsatzdosierung zur Herstellung von stabilen Zubereitungen, insbesondere von stabilen wässrigen kosmetischen Zubereitungen, verwenden.

Eine erfindungsgemäße Mischung bzw. eine erfindungsgemäße Zusammensetzung (wie nachfolgend definiert) ist vorzugsweise frei von Polyethylenglycol (PEG).

In eigenen Untersuchungen wurde unter anderem gefunden, dass Mischungen umfassend 1,2-Pentandiol, Natrium-Oleoylsarcosinat und Natrium-Laurylsulfoacetat weiter verbesserte lösungsvermittelnde Eigenschaften aufweisen im Vergleich zu Mischungen, die lediglich 1,2-Pentandiol und Natrium-Oleoylsarcosinat bzw. lediglich Pentan-1,2-diol und Natrium-Laurylsulfoacetat umfassen.

Wurde jedoch anstelle von 1,2-Pentandiol ein anderes organisches Di- oder Triol verwendet, wie beispielsweise Propylenglycol oder Glycerin, wurden schlechtere Ergebnisse erzielt. So waren die untersuchten Mischungen umfassend Propylenglycol, Natrium-Oleoylsarcosinat und Natrium-Laurylsulfoacetat bzw. Glycerin, Natrium-Oleoylsarcosinat und Natrium-Laurylsulfoacetat jeweils nicht klar und enthielten zudem noch ungelöste Feststoff-Bestandteile (siehe hierzu auch untenstehendes Beispiel 1).

Zudem wurde gefunden, dass Bestandteil (i), dabei insbesondere 1,2-Pentandiol, im Vergleich zu diversen anderen Glycolen wie 1,2-Propylenglycol bzw. im Vergleich zu Glycerin die lösungsvermittelnden Eigenschaften von Bestandteil (ii), dabei insbesondere Natrium-Oleoylsarcosinat bzw. von Bestandteil (iii), dabei insbesondere Natrium-Laurylsulfoacetat, oder Mischungen der Bestandteile (ii) und (iii) deutlich mehr verstärkt wird und dadurch merklich bessere Eigenschaften erzielt wurden.

Bevorzugt sind daher erfindungsgemäße Mischungen, die Bestandteile (i), (ii) und (iii) umfassen, dabei bevorzugt Pentan-1,2-diol, Natrium-Oleoylsarcosinat und Natrium-Laurylsulfoacetat, vorzugsweise in einer der vorstehend oder nachfolgend als bevorzugt bzw. besonders bevorzugt gekennzeichneten Ausgestaltungen, da mit diesen Mischungen besonders gute Ergebnisse im Sinne der vorliegenden Erfindung erzielt wurden.

Vorzugsweise umfasst eine erfindungsgemäße Mischung:
- 5 bis 30 Gew.-%, vorzugsweise 10 bis 20 Gew.-%, bevorzugt 12 bis 18 Gew.-%, besonders bevorzugt 13 bis 17 Gew.-% an Bestandteil (i), dabei vorzugsweise Pentan-1,2-diol,
- 5 bis 30 Gew.-%, vorzugsweise 10 bis 20 Gew.-%, bevorzugt 12 bis 18 Gew.-%, besonders bevorzugt 13 bis 17 Gew.-% an Bestandteil (ii), dabei vorzugsweise Natrium-Oleoylsarcosinat,
- optional 3 bis 22 Gew.-%, vorzugsweise 5 bis 20 Gew.-%, bevorzugt 8 bis 20 Gew.-%, besonders bevorzugt 11 bis 17 Gew.-% an Bestandteil (iii), dabei vorzugsweise Natrium-Laurylsulfoacetat,
wobei sich die Gewichtsprozentangaben jeweils auf die Gesamtmasse der Mischung beziehen.

Eine bevorzugte erfindungsgemäße Mischung umfasst oder besteht aus:
- 10 bis 20 Gew.-%, vorzugsweise 12 bis 18 Gew.-%, bevorzugt 13 bis 17 Gew.-%, besonders bevorzugt 14 bis 16 Gew.-% an Bestandteil (i), dabei vorzugsweise Pentan-1,2-diol,
- 10 bis 20 Gew.-%, vorzugsweise 12 bis 18 Gew.-%, bevorzugt 13 bis 17 Gew.-%, besonders bevorzugt 13 bis 16 Gew.-% an Bestandteil (ii), dabei vorzugsweise Natrium-Oleoylsarcosinat,
- 5 bis 20 Gew.-%, vorzugsweise 8 bis 20 Gew.-%, bevorzugt 11 bis 17 Gew.-%, besonders bevorzugt 12 bis 16,5 Gew.-% an Bestandteil (iii), dabei vorzugsweise Natrium-Laurylsulfoacetat,
- 25 Gew.-% oder mehr Wasser, vorzugsweise 30 bis 60 Gew.-% Wasser, bevorzugt 35 bis 55 Gew.-% Wasser,
- optional Natrium-Oleat, vorzugsweise 0,5 bis 5 Gew.-% Natrium-Oleat, bevorzugt 1 bis 3 Gew.-%,
- optional Natriumchlorid, vorzugsweise 1 bis 5 Gew.-% Natriumchlorid, bevorzugt 1,5 bis 4 Gew.-%,
- optional Dinatriumsulfoacetat, vorzugsweise 0,5 bis 3 Gew.-%, bevorzugt 0,6 bis 2 Gew.-%,
- optional Natriumsulfat, vorzugsweise 0,5 bis 3 Gew.-%, bevorzugt 1 bis 2 Gew.-%,
wobei sich die Gewichtsprozentangaben jeweils auf die Gesamtmasse der Mischung beziehen.

Eine besonders bevorzugte erfindungsgemäße Mischung umfasst oder besteht aus:
- 12 bis 18 Gew.-%, vorzugsweise 13 bis 17 Gew.-%, bevorzugt 14 bis 16 Gew.-% an Bestandteil (i), dabei vorzugsweise Pentan-1,2-diol,
- 12 bis 18 Gew.-%, vorzugsweise 13 bis 17 Gew.-%, bevorzugt 13 bis 16 Gew.-% an Bestandteil (ii), dabei vorzugsweise Natrium-Oleoylsarcosinat,
- 8 bis 20 Gew.-%, vorzugsweise 8 bis 18 Gew.-%, bevorzugt vorzugsweise 11 bis 17 Gew.-%, weiter bevorzugt 12 bis 16,5 Gew.-% an Bestandteil (iii), dabei vorzugsweise Natrium-Laurylsulfoacetat,
- 30 Gew.-% oder mehr Wasser, vorzugsweise 30 bis 60 Gew.-% Wasser, bevorzugt 35 bis 55 Gew.-% Wasser,
- optional Natrium-Oleat, vorzugsweise 0,5 bis 5 Gew.-% Natrium-Oleat, bevorzugt 1 bis 3 Gew.-%,
- Natriumchlorid, vorzugsweise 1 bis 5 Gew.-% Natriumchlorid, bevorzugt 1,5 bis 4 Gew.-%,
- Dinatriumsulfoacetat, vorzugsweise 0,5 bis 3 Gew.-%, bevorzugt 0,6 bis 2 Gew.-%,
- Natriumsulfat, vorzugsweise 0,5 bis 3 Gew.-%, bevorzugt 1 bis 2 Gew.-%,
wobei sich die Gewichtsprozentangaben jeweils auf die Gesamtmasse der Mischung beziehen.

Aufgrund der chemischen Zusammensetzung der erfindungsgemäßen Mischungen sind ihre physikalischen und chemischen Eigenschaften weitgehend bestimmt, gewisse Variationen sind aber nichtsdestoweniger möglich.

In einer bevorzugten Ausgestaltung weist eine erfindungsgemäße Mischung einen pH-Wert von 6 oder höher auf, vorzugsweise einen pH-Wert von 6,5 oder höher.

Bevorzugt liegt der pH-Wert einer erfindungsgemäßen Mischung im Bereich von 6 bis 7,5.

Es wurde gefunden, dass solche erfindungsgemäße Mischungen ein weiter verbessertes Lösungsvermögen und noch bessere lösungsvermittelnde Eigenschaften aufweisen, insbesondere im Vergleich zu Mischungen mit einem pH-Wert von kleiner als 6.

Die Einstellung des pH-Wertes einer erfindungsgemäßen Mischung kann beispielsweise durch Zugabe einer Base erfolgen, vorzugsweise einer anorganischen Base wie beispielsweise Alkalimetallhydroxiden oder Erdalkalimetallhydroxiden, dabei bevorzugt von NaOH oder KOH, wobei die anorganische Base vorzugsweise als wässrige Lösung zugegeben wird.

In einem weiteren Aspekt betrifft die vorliegende Erfindung eine Zusammensetzung zur Herstellung einer kosmetischen Zubereitung, vorzugsweise eines Körperpflegemittels, umfassend eine erfindungsgemäße Mischung, vorzugsweise in einer der vorgenannten bevorzugten Ausgestaltungen, sowie eine oder mehrere organische Substanzen mit einem log K_{OW} - Wert (wie oben definiert) im Bereich von 1 bis 12, vorzugsweise im Bereich von 2 bis 10, besonders bevorzugt im Bereich von 3 bis 8.

Die erfindungsgemäßen Mischungen erlauben die Herstellung transparenter Zusammensetzungen sowie transparenter kosmetischer Zubereitungen, wobei diese Zusammensetzungen bzw. Zubereitungen eine hervorragende Lagerstabilität aufweisen, bevorzugt über einen Zeitraum von 3 Monaten, insbesondere über einen Zeitraum von 6 Monaten.

Die Lagerstabilität der erfindungsgemäßen Mischungen und Zusammensetzungen zeigte sich daran, dass keine Entmischung oder Phasentrennung während des Lagerungszeitraumes von 3 bzw. bevorzugt 6 Monaten bei Tageslicht und bei konstanter Lagerungstemperatur zu beobachten war, vorzugsweise bei einer konstanten Lagerungstemperatur von 50°C, vorzugsweise bei einer konstanten Lagerungstemperatur von 40 °C, bevorzugt bei einer konstanten Lagerungstemperatur von 25 °C, insbesondere bevorzugt bei einer konstanten Lagerungstemperatur von 5 °C.

Daneben weisen erfindungsgemäße Mischungen und Zusammensetzungen eine hohe Stabilität auf, d.h. dass keine, keine wesentlichen bzw. lediglich in Ausnahmefällen geringe, nicht signifikante Veränderungen unter den oben genannten Lagerungsbedingungen und -zeiträumen bezüglich der Viskosität, des pH-Wertes, der Farbe und des Geruchs beobachtet werden.

Vorzugsweise weist eine erfindungsgemäße Zusammensetzung eine sehr geringe Trübung auf. Die Trübung wird gemessen in NTU (nephelometric turbidity units).

Eine erfindungsgemäß bevorzugte Zusammensetzung ist transparent, und weist vorzugsweise eine Trübung von weniger als 10 NTU auf, bevorzugt von weniger als 5 NTU, besonders bevorzugt von weniger als 3 NTU, jeweils bestimmt gemäß DIN ISO EN 27027.

Die Trübung im Rahmen der vorliegenden Erfindung wird gemessen nach DIN ISO EN 27027 mit 90° IR-Streulicht.

Die Messung der Trübung kann beispielsweise mit einem Hach 2100N IS Labortrübungsmessgerät mit 860 nm (Infrarot) LED oder mit einem Labor-Trübungs-Photometer NEPHLA der Dr. Bruno Lange GmbH & CO. KG erfolgen.

Die Lagerstabilität einer transparenten erfindungsgemäßen Zusammensetzungen zeigte sich daran, dass diese während des Lagerungszeitraumes von 3 bzw. bevorzugt 6 Monaten bei Tageslicht und bei konstanter Lagerungstemperatur transparent blieb, vorzugsweise bei einer konstanten Lagerungstemperatur von 50°C, vorzugsweise bei einer konstanten Lagerungstemperatur von 40 °C, bevorzugt bei einer konstanten Lagerungstemperatur von 25 °C, insbesondere bevorzugt bei einer konstanten Lagerungstemperatur von 5°C.

Eine erfindungsgemäß weiter bevorzugte Zusammensetzung ist dadurch gekennzeichnet, dass die Gesamtmenge der organischen Substanz(en) mit einem log K_{OW} - Wert im Bereich von 1 bis 12 in einem Bereich von 0,1 bis 5 Gew.-%, vorzugsweise in einem Bereich von 0,1 bis 3 Gew.-%, bevorzugt in einem Bereich von 0,15 bis 1 Gew.-%, besonders bevorzugt in einem Bereich von 0,2 bis 0,8 Gew.-%, jeweils bezogen auf die Gesamtmasse der Zusammensetzung liegt.

Dabei bevorzugte organischen Substanz(en) weisen vorzugsweise einen log K_{OW} - Wert im Bereich von 2 bis 10 auf, besonders bevorzugt im Bereich von 3 bis 8, und sind vorzugsweise ausgewählt aus der Gruppe der zum Einsatz in kosmetischen Zubereitungen, vorzugsweise in Körperpflegemitteln, geeigneten Substanzen. Solche bevorzugten Substanzgruppen, die entsprechende log K_{OW} - Werte aufweisen sind Parfümöle, Riechstoffe, etherische Öle, Wirkstoffe, Antioxidantien, Kühlwirkstoffe, Deodorantwirkstoffe, UV-Filter, Vitamine, hautaufhellende Wirkstoffe und antiirritierende Wirkstoffe.

In eigenen Untersuchungen wurden als organische Substanzen stellvertretend die nachfolgenden Substanzen eingesetzt aus der Gruppe der Kühlwirkstoffe, Hilfsstoffe, etherischen Öle und Parfümöle etc.:
Frescolat^{®} ML (Menthyllactat), Salbeiöl (Salvia Officinalis Oil), Avocadoöl (Persea Gratissima Oil), Mandelöl (Prunus Amygdalus Dulcis Oil), Rosenöl (Rosa Damascena Flower Oil), verschiedene Parfümöle (mit den Namen Lait de Rose, Lait Vitamine Sun, L'Eau d'été, Jasmin Flowers), Bisabolol, Farnesol, Crinipan^{®} AD (Schuppenwirkstoff Climbazol), Cetearyl Ethylhexanoat, Paraffinöl (Paraffinum Liquidum) und Dragocid^{™} Liquid (Konservierungsmittel-Gemisch aus Phenoxyethanol, Methylparaben, Ethylparaben, Butylparaben, Propylparaben und Isobutylparaben).

Eine erfindungsgemäß weiter bevorzugte Zusammensetzung ist dadurch gekennzeichnet, dass das Gewichtsverhältnis der Gesamtmenge von Bestandteil (i), dabei vorzugsweise Pentan-1,2-diol, und Bestandteil (ii), dabei vorzugsweise Natrium-Oleoylsarcosinat, zu der Gesamtmenge an Substanzen mit einem log K_{OW} - Wert im Bereich von 1 bis 12 größer als 0,5 ist, vorzugsweise größer als 0,75, bevorzugt größer als 1, besonders bevorzugt größer als 1,25.

Eine erfindungsgemäße Zusammensetzung zeichnet sich unter anderem aus durch:
- eine gute Viskositätsstabilität,
- eine gute pH-Stabilität,
- eine gute Temperaturstabilität,
- eine gute Farbstabilität,
- eine gute Geruchsstabilität.

In einem weiteren Aspekt betrifft die Erfindung eine kosmetische Zubereitung, vorzugsweise ein Körperpflegemittel, umfassend
- eine erfindungsgemäße Mischung, vorzugsweise in einer der der als bevorzugt gekennzeichneten Ausgestaltungen, sowie eine oder mehrere organische Substanzen mit einem log K_{OW} - Wert im Bereich von 1 bis 12, vorzugsweise im Bereich von 2 bis 10, besonders bevorzugt im Bereich von 3 bis 8
   oder
   eine erfindungsgemäße Zusammensetzung, vorzugsweise in einer der der als bevorzugt gekennzeichneten Ausgestaltungen
sowie
- gegebenenfalls weitere kosmetisch wirksame Bestandteile, vorzugsweise für die Körperpflege wirksame Bestandteile,
- gegebenenfalls sonstige Bestandteile.

Erfindungsgemäße kosmetische Zubereitungen sind vorzugsweise transparent und weisen insbesondere eine Trübung von weniger als 10 NTU (nephelometric turbidity units) auf gemäß DIN ISO EN 27027.

Ferner bewirkt eine erfindungsgemäße Mischung, eine erfindungsgemäße Zusammensetzung bzw. einer erfindungsgemäße kosmetische Zubereitung ein sehr gutes Feuchthaltvermögen der Haut (Moisturizing), wirkt also auch der Austrocknung der Haut entgegen.

Substanzen und Hilfsstoffe, welche eine erfindungsgemäße Zusammensetzung oder kosmetische Zubereitung enthaltend eine erfindungsgemäße Mischung zusätzlich enthalten kann, sind beispielsweise:
Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, plastifizierende Mittel, deckfähige Mittel, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-betadicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Aromen, Geschmackstoffe sowie Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten, Alkohole und (weitere) Polyole, vorzugsweise die in WO 2005/123101 genannten, weitere Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten.

Bevorzugte flüssige Trägerstoffe, die Bestandteil einer erfindungsgemäßen kosmetischen Zubereitung sein können, sind ausgewählt aus der Gruppe bestehend aus Glycerin, 1,2-Propylenglycol, 1,2-Butylenglycol, 1,3-Butylenglycol, 1,2-Decandiol und Ethanol sowie Mischungen zweier oder mehrerer der genannten flüssigen Trägerstoffe.

In Abhängigkeit von dem jeweiligen Produkttyp einer erfindungsgemäßen kosmetischen Zubereitung kann der Anteil an flüssigen Trägerstoffen beträchtlich variieren. Vorzugsweise liegt die Gesamtmenge an Glycerin, 1,2-Propylenglycol, 1,2-Butylenglycol und Ethanol im Bereich von 1 bis 50 Gew.-%, bevorzugt im Bereich von 3 bis 40 Gew.-%, bezogen auf das Gesamtgewicht der erfindungsgemäßen Zubereitung.

Vorzugsweise liegt die Gesamtmenge an Wasser in einer erfindungsgemäßen kosmetischen Zubereitung im Bereich von 20 bis 90 Gew.-%, bevorzugt im Bereich von 30 bis 85 Gew.-%, weiter bevorzugt im Bereich von 40 bis 80 Gew.-%, bezogen auf das Gesamtgewicht der Zubereitung.

Darüber hinaus können die Alkohole des Bestandteils (i), insbesondere Pentan-1,2-diol, zusätzlich zur Verbesserung der Hautfeuchtigkeit und/oder aufgrund der konservierenden Eigenschaften in einer Gesamtmenge von bis zu 10 Gew.-% in erfindungsgemäßen kosmetischen Zubereitungen enthalten sein. Diese Menge versteht sich einschließlich der Menge an Bestandteil (i), die als Bestandteil einer erfindungsgemäßen Mischung bzw. Zusammensetzung in eine erfindungsgemäße kosmetische Zubereitung eingebracht wird.

Bevorzugte feste Trägerstoffe, die Bestandteil einer erfindungsgemäßen Zubereitung sein können, sind Hydrokolloide wie Stärken, abgebaute Stärken, chemisch oder physikalisch modifizierte Stärken, Dextrine, (pulverförmige) Maltodextrine (bevorzugt mit einem Dextroseequivalent-Wert von 5 bis 25, bevorzugt von 10 - 20), Lactose, Siliciumdioxid, Glucose, modifizierte Cellulosen, Gummi Arabicum, Ghatti-Gum, Traganth, Karaya, Carrageenan, Pullulan, Curdlan, Xanthan Gum, Gellan Gum, Guarkernmehl, Johannisbrotkernmehl, Alginate, Agar, Pektin und Inulin sowie Mischungen zweier oder mehrerer dieser Feststoffe, insbesondere Maltodextrine (bevorzugt mit einem Dextroseequivalent-Wert von 15-20), Lactose, Siliciumdioxid und/oder Glucose.

Die jeweils einzusetzenden Mengen an kosmetischen Hilfs- und Trägerstoffen können in Abhängigkeit von der Art der jeweiligen kosmetischen Zubereitung vom Fachmann leicht ermittelt werden.

Vorzugsweise enthalten erfindungsgemäßen Zubereitungen ein oder mehrere Konservierungsmittel oder ein oder mehrere Antioxidantien.

Die Menge der Antioxidantien (eine oder mehrere Verbindungen) in den Zubereitungen beträgt vorzugsweise 0,001 - 10 Gew.-%, besonders bevorzugt 0,05 - 5 Gew.-%, insbesondere 0,1 - 3 Gew.-%, bezogen auf das Gesamtgewicht der Zubereitung.

Vorteilhaft werden die Antioxidantien aus der folgenden Gruppe gewählt: Aminosäuren (z.B. Glycin, Histidin, 3,4-Dihydroxyphenylalanin, Tyrosin, Tryptophan) und deren Derivate, Imidazole (z.B. Urocaninsäure) und deren Derivate, Peptide (D,L-Carnosin, D-Carnosin, L-Carnosin, Anserin) und deren Derivate, Carotinoide, Carotine (z.B. alpha-Carotin, beta-Carotin, Lycopin) und deren Derivate, Chlorogensäure und deren Derivate, Liponsäure und deren Derivate, Aurothioglucose, Propylthiouracil und andere Thiole (z.B. Thioredoxin, Glutathion, Cystein, Cystin, Cystamin und deren Glycosyl- und N-Acylderivate oder deren Alkylester) sowie deren Salze, Dilaurylthiodipropionat, Distearylthiodipropionat, Thiodipropionsäure und deren Derivate sowie Phenolsäureamide phenolischer Benzylamine (z.B. Homovanillinsäure-, 3,4-Dihydroxyphenylessigsäure-, Ferulasäure-, Sinapinsäure-, Kaffeesäure-, Dihydroferulasäure-, Dihydrokaffeesäure-, Vanillomandelsäure- oder 3,4-Dihydroxymandelsäureamide des 3,4-Dihydroxybenzyl-, 2,3,4-Trihydroxybenzyl- bzw. 3,4,5-Trihydroxybenzylamins), Catecholoxime oder Catecholoximether (z.B. 3,4-Dihydroxybenzaldoxim oder 3,4-Dihydroxybenzaldehyd-O-ethyloxim), 2-Hydrazino- 1,3thiazole und Derivate, ferner (Metall-)chelatoren (z.B. 2-Hydroxyfettsäuren, Phytinsäure, Lactoferrin), Huminsäure, Gallensäuren, Gallenextrakte, Bilirubin, Biliverdin, Folsäure und deren Derivate, Ubichinon und Ubichinol und deren Derivate, Vitamin C und dessen Derivate (z.B. Ascorbylpalmitat, Magnesiumascorbylphosphat, Ascorbylacetat), Tocopherole und Derivate (z.B. Vitamin-E-Acetat), Vitamin A und Derivate (z.B. Vitamin-A-Palmitat), Rutinsäure und deren Derivate, Flavonoide (z.B. Quercetin, alpha-Glucosylrutin) und deren Derivate, Phenolsäuren (z.B. Gallussäure, Ferulasäure) und deren Derivate (z.B. Gallussäurepropylester, -ethylester, -octylester), Furfurylidenglucitol, Dibutylhydroxytoluol, Butylhydroxyanisol, Harnsäure und deren Derivate, Mannose und deren Derivate, Zink und dessen Derivate (z.B. ZnO, ZnSO₄), Selen und dessen Derivate (z.B. Selenomethionin), Stilbene und deren Derivate (z.B. Stilbenoxid, Resveratrol).

Sofern Vitamin E bzw. Vitamin-E-Derivate als Antioxidantien eingesetzt werden, liegt deren Gesamtkonzentration vorzugsweise in dem Bereich von 0,01 - 5 Gew.-%, bevorzugt im Bereich von 0,1 - 3 Gew.-%, bezogen auf das Gesamtgewicht der erfindungsgemäßen kosmetischen Zubereitung.

Sofern Vitamin A bzw. Vitamin-A-Derivate, oder Carotine bzw. deren Derivate als Antioxidantien eingesetzt werden, liegt deren Gesamtkonzentration vorzugsweise in dem Bereich von 0,01 - 5 Gew.-%, bevorzugt im Bereich von 0,1 - 3 Gew.-%, bezogen auf das Gesamtgewicht der erfindungsgemäßen kosmetischen Zubereitung.

Der eine bzw. die mehreren weiteren Stoffe mit physiologischer Kühlwirkung, die als Bestandteil in einer erfindungsgemäßen Zusammensetzung oder einer erfindungsgemäßen kosmetischen Zubereitung eingesetzt werden können, werden dabei vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (1-Menthoxy)-1,2-propandiol, (I-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Mischungen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (dabei vorzugsweise Menthancarbonsäure-N-ethylamid [WS3] oder N^{α}-(Menthancarbonyl)glycinethylester [WS5], wie in US 4,150,052 beschrieben, Menthancarbonsäure-N-(4-cyanophenyl)amid oder Menthancarbonsäure-N-(4-cyanomethylphenyl)amid wie in WO 2005/049553 beschrieben, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol) oder Cubebol.

In einer bevorzugten Ausführungsform enthält eine erfindungsgemäße kosmetische Zubereitung mindestens einen UVA-Filter und/oder mindestens einen UVB-Filter und/oder mindestens ein anorganisches Pigment. Die Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Spray bilden.

Erfindungsgemäße Zubereitungen im Bereich der Kosmetika, die eine erfindungsgemäße Mischung oder eine erfindungsgemäße Zusammensetzung (jeweils wie oben definiert) enthalten, werden besonders vorteilhaft mit Substanzen kombiniert, die UV-Strahlung absorbieren oder reflektieren, namentlich für kosmetische oder hautschützende Zwecke, wobei die Gesamtmenge der Filtersubstanzen von 0,01 bis 30 Gew.-%, vorzugsweise 0,1 bis 20 Gew.-%, insbesondere 1 bis 15 Gew.-% beträgt, bezogen auf das Gesamtgewicht der Zubereitungen, um kosmetische Zubereitungen zur Verfügung zu stellen, die das Haar bzw. die Haut vor ultravioletter Strahlung schützen. Vorteilhaft enthalten die erfindungsgemäßen kosmetischen Zubereitungen mindestens einen UVA-Filter und/oder mindestens einen UVB-Filter und/oder mindestens ein anorganisches Pigment, so dass ein Lichtschutzfaktor von zumindest > 2 (bevorzugt > 5) erreicht wird.

Vorteilhafte anorganische Lichtschutzpigmente sind feindisperse Metalloxide und Metallsalze die ebenfalls in WO 2005/123101 genannt sind. Die Gesamtmenge an anorganischen Pigmenten, insbesondere hydrophoben anorganischen Mikropigmenten in einer erfindungsgemäßen kosmetischen Zubereitung liegt vorzugsweise im Bereich von 0,1 bis 20 Gew.-%, bevorzugt im Bereich von 0,5 bis 15 Gew.-%, bezogen auf das Gesamtgewicht der Zubereitung.

Besonders vorteilhaft ist eine Kombination der erfindungsgemäßen Mischungen bzw. Zusammensetzungen mit UV-Filtern (UV/A-, UV/B- und Breitbandfiltern) (Lichtschutzmitteln). Der Einsatz der erfindungsgemäßen Mischungen hat co-emulgierende Eigenschaften und führt dabei regelmäßig zu einer Verbesserung der Lagerstabilität, einer Erhöhung der UVA und/oder UVB-Lichtschutzfaktoren (SPF) und der Wasserfestigkeit entsprechender Sonnenschutzprodukte.

Eine erfindungsgemäße Mischung bzw. Zusammensetzung wird in einer bevorzugten Ausgestaltung zusammen mit Lichtschutzmitteln formuliert. Bevorzugte Lichtschutzmittel sind z.B. organische UV-Absorber aus der Klasse der 4-Aminobenzoesäure und Derivate, Salicylsäure-Derivate, Benzophenon-Derivate, Dibenzoylmethan-Derivate, Diphenylacrylate, 3-Imidazol-4-yl-acrylsäure und deren Ester, Benzofuran-Derivate, Benzylidenmalonat-Derivate, polymere UV-Absorber (enthaltend einen oder mehrere Silicium-organische Reste), Zimtsäure-Derivate, Campher-Derivate, Trianilino-s-Triazin-Derivate, 2-Hydroxyphenylbenzotriazol-Derivate, 2-Phenylbenzimidazol-5-sulfonsäure und deren Salze, Anthranilsäurementhylester, Benzotriazolderivate.

Die Gesamtmenge aller wasserlöslichen UV-Filtersubstanzen in einer bevorzugten erfindungsgemäßen kosmetischen Zubereitung liegt vorzugsweise im Bereich von 0,1 bis 12 Gew.-%, bevorzugt 0,5 bis 8 Gew.-%, bezogen auf das Gesamtgewicht der erfindungsgemäßen Zubereitung.

Dabei bevorzugt sind (einfach oder mehrfach) sulfonierte wasserlösliche UV-Filter, welche bevorzugt gewählt sind der Gruppe bestehend aus Phenylen-bis-benzimidazyl-tetrasulfonsäure-dinatriumsalz bzw. deren Salzen und/oder die entsprechende Disulfonsäure bzw. deren Salzen und/oder 2-Phenylbenzimidazol-5-sulfonsäure bzw. deren Salzen und/oder 2-Hydroxy-4-methoxybenzophenon-5-sulfonsäure bzw. deren Salzen und/oder 4-(2-Oxo-3-bornylidenmethyl)-benzolsulfonsäure bzw. deren Salzen und/oder 2-Methyl-5-(2-oxo-3-bornyliden-methyl)-benzolsulfonsäure bzw. deren Salzen und/oder Benzol-1,4-di-(2-oxo-3-bornylidenmethyl)-10-sulfonsäure bzw. deren Salzen.

Vorteilhafte UV-Filter und anorganische Lichtschutzpigmente sind genannt in WO 2005/123101. Besonders zur Kombination geeignete UV-Absorber sind ebenfalls genannt in WO 2005/123101.

Besonders geeignete UV-Filter sind dabei
- p-Aminobenzoesäure
- 3-(4'-Trimethylammonium)-benzyliden-bornan-2-on-methylsulfat
- Salicylsäure-homomenthylester (Neo Heliopan^{®}HMS)
- 2-Hydroxy-4-methoxy-benzophenon (Neo Heliopan^{®}BB)
- 2-Phenylbenzimidazolsulfonsäure (Neo Heliopan^{®}Hydro)
- Terephthalyliden-dibornansulfonsäure und Salze (Mexoryl^{®}SX)
- 4-tert.-Butyl-4'-methoxydibenzoylmethan (Neo Heliopan^{®}357)
- 3-(4'-Sulfo)benzyliden-bornan-2-on und Salze
- 2-Ethylhexyl-2-cyano-3,3-diphenylacrylat (Neo Heliopan^{®}303)
- N-[(2 und 4)-[2-(oxoborn-3-yliden)methyl]benzyl]-acrylamid-Polymer
- p-Methoxyzimtsäure-2-ethylhexylester (Neo Heliopan^{®}AV)
- p-Aminobenzoesäure-ethylester (25 Mol) ethoxyliert
- p-Methoxyzimtsäure-isoamylester (Neo Heliopan^{®}E1000)
- 2,4,6-Trianilino-(p-carbo-2'-ethylhexyl-1'-oxy)-1,3,5-triazin (Uvinul^{®}T150)
- Phenol, 2-(2H-benzotriazol-2-yl)-4-methyl-6-(2-methyl-3(1,3,3,3-tetramethyl-1-(trimethylsilyl)-oxy)-disiloxyanyl)-propyl) (Mexoryl^{®}XL)
- 4,4'-[(6-[4-(1,1-Dimethyl)-aminocarbonyl)-phenylamino]-1,3,5-triazin-2,4-diyl)-diimino]-bis-(benzoesäure-2-ethylhexylester) (Uvasorb HEB)
- 3-(4'-Methylbenzyliden)-d,l-campher (Neo Helipan^{®}MBC)
- 3-Benzylidencampher
- Salicylsäure-2-ethylhexylester (Neo Helipan^{®}OS)
- 4-Dimethylaminobenzoesäure-2-ethylhexylester (Padimate O)
- Hydroxy-4-methoxy-benzophenon-5-sulfonsäure und Na-Salz
- 2,2'-Methylen-bis-(6-(2H-benztriazol-2-yl)-4-1,1,3,3-tetramethylbutyl)-phenol) (Tinosorb^{®}M)
- Phenylen-bis-benzimidazyl-tetrasulfonsäure-dinatriumsalz (Neo Heliopan^{®}AP)
- 2,4-Bis-[{(4-(2-Ethyl-hexyloxy)-2-hydroxy}-phenyl]-6-(4-methoxyphenyl)-1,3,5-triazin (Tinosorb^{®}S)
- Benzylidenmalonat-Polysiloxan (Parsol^{®}SLX)
- Menthylanthranilat (Neo Heliopan^{®}MA)
- 2-(4-Diethylamino-2-hydroxybenzoyl)-benzoesäure-hexylester (Uvinul^{®} A Plus)
- Indanylidenverbindungen gemäß WO 02/38537

Eine erfindungsgemäße Mischung bzw. Zusammensetzung kann ferner in kosmetische und/oder dermatologische Zubereitungen eingearbeitet werden, die Pigmente, bevorzugt feinteilige Pigmente enthalten. Hierbei kann es sich um organische oder anorganische Pigmente handeln. Bevorzugtes organisches Pigment ist 2,2'-Methylen-bis-[6-(2H-benzotriazol-2-yl)-4-(1,1,3,3-tetramethylbutyl)-phenol] (Tinosorb^{®} M). Geeignete anorganische Pigmente oder Mikropigmente auf Basis von Metalloxiden und/oder anderen in Wasser schwerlöslichen oder unlöslichen Metallverbindungen sind insbesondere Oxide des Titans (TiO₂) Zinks (ZnO), Eisens (z.B. Fe₂O₃), Zirkoniums (ZrO₂) Siliciums (SiO₂) Mangans (z.B. MnO), Aluminiums (Ai₂O₃), Cers (z.B. Ce₂O₃), Mischoxiden der entsprechenden Metalle sowie Abmischungen aus solchen Oxiden. Diese Pigmente sind röntgenamorph oder nichtröntgenamorph. Besonders bevorzugt sind feinteilige Pigmente auf der Basis von TiO₂ und ZnO.

Erfindungsgemäße kosmetische Zubereitungen enthalten in machen Ausgestaltungen einen oder mehrere (Metall)-Chelatoren. Bevorzugt einzusetzende (Metall)-Chelatoren sind dabei die in WO 2005/123101 genannten Verbindungen.

Die Menge der Antiirritantien (eine oder mehrere Verbindungen) in einer erfindungsgemäßen kosmetischen Zubereitung beträgt bevorzugt 0,0001 - 10 Gew.-%, insbesondere 0,001 - 5 Gew.-%, bezogen auf das Gesamtgewicht der Zubereitung.

Eine erfindungsgemäße Mischung oder eine erfindungsgemäße Zusammensetzung (jeweils wie oben definiert) kann insbesondere in erfindungsgemäßen kosmetischen Zubereitungen vorteilhaft in Kombination mit Insektrepellents wie z.B. DEET, IR 3225 oder Dragorepel^{™} (Symrise) eingesetzt werden.

Eine erfindungsgemäße Mischung oder eine erfindungsgemäße Zusammensetzung (jeweils wie oben definiert) kann insbesondere in kosmetischen erfindungsgemäßen Zubereitungen vorteilhaft in Kombination mit Haarpflegemitteln und Antischuppenwirkstoffen (z.B. Climbazol, Ketoconazol, Piroctonoleamin, Zink-Pyrithion) eingesetzt werden.

Auch kann eine erfindungsgemäße Mischung oder eine erfindungsgemäße Zusammensetzung (jeweils wie oben definiert) in zahlreichen Fällen vorteilhaft in Kombination mit einem oder mehreren Konservierungsmitteln in erfindungsgemäßen Zubereitungen eingesetzt werden. Vorzugsweise gewählt werden hierbei die in WO 2005/123101 genannten Konservierungsmittel.

Erfindungsgemäßen kosmetische Zubereitungen können auch für kosmetische Zwecke verwendbare Pflanzenextrakte enthalten. Vorzugsweise werden die Pflanzenextrakte gewählt aus der auf Seite 44 der 3. Auflage des Leitfadens zur Inhaltsstoffdeklaration kosmetischer Mittel, herausgegeben vom Industrieverband Körperpflegemittel und Waschmittel e.V. (IKW), Frankfurt, beginnenden Tabelle aufgeführten Stoffe. Vorteilhaft sind ferner insbesondere die in WO 2005/123101 genannten Extrakte.

Erfindungsgemäße kosmetische Zubereitungen, können, insbesondere wenn kristalline oder mikrokristalline Festkörper wie beispielsweise anorganische Mikropigmente in die Zubereitungen eingearbeitet werden sollen, zusätzlich auch in WO 2005/123101 genannte anionische, kationische, nichtionische und/oder amphotere Tenside enthalten.

Als weitere Stabilisierungsmittel können erfindungsgemäße kosmetische Zubereitungen zusätzlich Hydrogelbildner wie z.B. Carbomere, Acrylat-Crosspolymere, Xantane, Alginate usw. enthalten.

Die Lipidphase kann vorzugsweise ein oder mehrere Substanzen umfassen. gewählt aus den folgenden Substanzgruppen:
- Mineralöle, Mineralwachse,
- Öle, wie Triglyceride der Caprin- oder der Caprylsäure, ferner natürlicher Öle wie z.B. Rizinusöl,
- Fette, Wachse und andere natürliche und synthetische Fettkörper, vorzugsweise Ester von Fettsäuren mit Alkoholen niedriger C-Zahl (<10), z.B. mit Isopropanol, Propylenglycol oder Glycerin, oder Ester von Fettalkoholen mit Alkansäuren niedriger C-Zahl (<10) oder mit Fettsäuren,
- Alkylbenzoate,
- Silikonöle und Siloxane, dabei vorzugsweise Dimethylpolysiloxan, Diethylpolysiloxan, Diphenylpolysiloxan sowie Mischformen daraus, Cyclomethicon (Octamethylcyclotetrasiloxan), Hexamethylcyclotrisiloxan, Polydimethylsiloxan und Poly(methylphenylsiloxan).

Die Lipidphase einer erfindungsgemäßen kosmetischen Zubereitung kann vorzugsweise gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, -palmitat, -stearat, -oleat, n-Butylstearat, n-Hexyllaurat, n-Decyloleat, Isooctylstearat, Isononylstearat, Isononylisononanat, 2-Ethyl-hexylpalmitat, Ethylhexyllaurat, 2-Hexyldecylstearat, 2-Octyldodecylpalmitat, Oleyloleat, Oleylerucat, Erucyloleat sowie synthetische, halbsynthetische und natürliche Gemische solcher Ester, z.B. Jojobaöl, 2-Ethylhexyl-2-Ethylhexanoat (z.B. Dragoxat EH), Ethylhexyl Isononanoat (z.B: Dragoxat 89), Cetearyl-2-Ethylhexanoat, Diisopropyladipat, Triisonananoin.

Wesentliche Anwendungsgebiete für die erfindungsgemäßen Mischungen und Zusammensetzungen sind kosmetische, insbesondere wässrige kosmetische Zubereitungen, die (abgesehen von der erfindungsgemäßen Mischung) wie üblich zusammengesetzt sind und dem kosmetischen Lichtschutz, zur Behandlung, der Pflege und der Reinigung der Haut und/oder der Haare dienen.

Zur Anwendung werden die erfindungsgemäßen kosmetischen Zubereitungen in der für Kosmetika üblichen Weise auf die Haut und/oder die Haare in ausreichender Menge aufgebracht. Entsprechend können erfindungsgemäße kosmetische Zubereitungen, je nach ihrem Aufbau, vorzugsweise verwendet werden als Hautpflegemittel, z.B. Hautschutzcreme, Tages- oder Nachtcreme, Sonnenschutzmittel, der After-sun-Präparat (z.B. After-sun-Lotion) Pflegemaske, Gel-Pads, Gesichtswasser, feuchte Pflege- und/oder Tränkungslösung, z.B. für kosmetische Tücher, Reinigungstücher, Reinigungsseife, Schaum- oder Duschbad, Flüssigseife, Seifenstück, Shampoo (z.B. 2-in-1 Shampoo, Anti-Schuppen-Shampoo, Babyshampoo, Shampoo für trockene Kopfhaut, Shampookonzentrat), Haarpflegemittel, Haarconditioner, Haarcoloration, Haarspülung, Haarstylingmittel (z.B. Haargel), Deodorant, Antitranspirans (z.B. Roll-on oder Stick), Rasierpräparat (z.B. Aftershave Balm, Preshave- oder Aftershave-Lotion) oder als Make-Up Entferner.

Dabei liegt eine erfindungsgemäße kosmetische Zubereitung in Form einer Emulsion, Lotion, Fluid, Creme, Mikroemulsion, Gel (z.B. Hydro- oder Hydrodispersionsgel), Balsam, Pumpspray, alkoholische oder wässrig/alkoholische Lösung vor.

In einem weiteren Aspekt betrifft die Erfindung eine kosmetische Zubereitung, wobei die kosmetische Zubereitung ein Körperpflegemittel ist, welches vorzugsweise ausgewählt ist aus der Gruppe bestehend aus wässrigen und wässrig-alkoholischen Systemen wie Duschgelen, Shampoos, alkoholischen Parfüms, wässrig- alkoholische Parfüms, After Shave Gelen, Cremes, Lotionen, Haarpflegemitteln und Haarstylingmitteln.

Eine erfindungsgemäß bevorzugte kosmetische Zubereitung ist dadurch gekennzeichnet, dass die kosmetische Zubereitung, vorzugsweise das Körperpflegemittel,
- 0,1 bis 15 Gew.-%, bevorzugt 0,5 bis 7 Gew.-%, weiter bevorzugt 0,5 bis 5 Gew.-%, und besonders bevorzugt 1 bis 3 Gew.-% einer erfindungsgemäßen Mischung, vorzugsweise in einer der als bevorzugt gekennzeichneten Ausgestaltungen,
oder
- 0,1 bis 15 Gew.-.%, bevorzugt 0,5 bis 7 Gew.-%, weiter bevorzugt 0,5 bis 5 Gew.-%, und besonders bevorzugt 1 bis 3 Gew.-% einer erfindungsgemäßen Zusammensetzung, vorzugsweise in einer der als bevorzugt gekennzeichneten Ausgestaltungen,
umfasst, jeweils bezogen auf die Gesamtmasse der kosmetischen Zubereitung.

Die jeweils einzusetzende Menge an erfindungsgemäßer Mischung bzw. erfindungsgemäßer Zusammensetzung kann in Abhängigkeit von der Art der jeweiligen kosmetischen Zubereitung, auch in Abhängigkeit von deren Einsatzzweck, deren Viskosität sowie den vorhandenen weiteren Wirk-, Hilfs- und/oder Trägerstoffen, vom Fachmann leicht ermittelt werden.

In einem weiteren Aspekt betrifft die Erfindung ein Verfahren zur Erhöhung der Löslichkeit einer organischen Substanz und/oder zur Verringerung der Trübung einer Zubereitung, enthaltend eine organische Substanz und/oder zur Erhöhung der Transparenz einer Zubereitung enthaltend eine oder mehrere organische Substanzen, wobei die organische Substanz jeweils einen log K_{OW} - Wert im Bereich von 1 bis 12 aufweist, vorzugsweise im Bereich von 2 bis 10, besonders bevorzugt im Bereich von 3 bis 8,
umfassend den Schritt:
- Vermischen der organischen Substanz(en) mit einer erfindungsgemäßen Mischung, vorzugsweise in einer der als bevorzugt gekennzeichneten Ausgestaltungen.

In einem weiteren Aspekt betrifft die Erfindung ein Verfahren zum Herstellen einer erfindungsgemäßen Mischung, einer erfindungsgemäßen Zusammensetzung oder einer erfindungsgemäßen kosmetischen Zubereitung, jeweils vorzugsweise in einer der als bevorzugt gekennzeichneten Ausgestaltungen, umfassend den Schritt:
- Mischen von Bestandteil (i) und Bestandteil (ii) sowie gegebenenfalls Bestandteil (iii).

In einer bevorzugten Ausgestaltung wird das erfindungsgemäße Verfahren zur Herstellung einer kosmetischen Zubereitung so ausgeführt, dass sich durch das Vermischen die organische(n) Substanz(en) in einer erfindungsgemäßen Mischung löst bzw. lösen, wobei vorzugsweise eine erfindungsgemäße Zusammensetzung in Form einer Lösung entsteht.

Für die bevorzugten organischen Substanz(en) gilt das oben Gesagte hier entsprechend.

Ferner betrifft die vorliegend Erfindung die Verwendung eines 1,2-Alkandiols mit 5 bis 8 C-Atomen, dabei vorzugsweise von 1,2-Pentandiol, oder einer Mischung von 1,2-Alkandiolen mit 5 bis 8 C-Atomen, dabei vorzugsweise einer Mischung enthaltend 1,2-Pentandiol, zur Verbesserung der lösungsvermittelnden Wirkung, insbesondere der Lösefähigkeit, von Tensiden.

Bevorzugte Tenside sind die als bevorzugt gekennzeichneten Verbindungen der Bestandteile (ii) und/oder (iii) einer erfindungsgemäßen Mischung, dabei insbesondere Natrium-Oleoylsarcosinat, Natrium-Laurylsulfoacetat oder deren Mischung, wobei wiederum bevorzugt eine Mischung von Natrium-Oleoylsarcosinat und Natrium-Laurylsulfoacetat im Gewichtsverhältnis von 10 : 1 bis 1 : 4 ist, bevorzugt im Gewichtsverhältnis von 4 : 1 bis 1 : 2.

Erfindungsgemäß besonders bevorzugte Ausgestaltungen:
Ausgestaltung 1: Verwendung von Pentan-1,2-diol in Kombination mit einer Verbindung aus der Gruppe bestehend aus Natrium-Oleoylsarcosinat und Natrium-Laurylsulfoacetat und deren Mischung
   - als Lösungsvermittler für eine oder mehrere organische Substanzen,
   - zur Erhöhung der Löslichkeit einer oder mehrerer organischer Substanzen,
   - zur Verringerung der Trübung einer Zubereitung, vorzugsweise einer Dispersion und/oder Emulsion, enthaltend eine oder mehrere organische Substanzen,
   und/oder
   - zur Erhöhung der Transparenz einer Zubereitung, vorzugsweise einer Dispersion und/oder Emulsion, enthaltend eine organische Substanz,
   wobei die organische Substanz jeweils vorzugsweise einen log K_{OW} - Wert im Bereich von 1 bis 12 aufweist, bevorzugt im Bereich von 2 bis 10, besonders bevorzugt im Bereich von 3 bis 8.
Ausgestaltung 2: Verwendung nach Ausgestaltung 1, dadurch gekennzeichnet, dass die organische Substanz einen log K_{OW} - Wert im Bereich von 1 bis 12 aufweist, vorzugsweise im Bereich von 2 bis 10, besonders bevorzugt im Bereich von 3 bis 8, und ausgewählt ist aus der Gruppe der zum Einsatz in kosmetischen Zubereitungen, vorzugsweise in Körperpflegemitteln, geeigneten Substanzen.
Ausgestaltung 3: Mischung umfassend Pentan-1,2-diol und Natrium-Oleoylsarcosinat sowie gegebenenfalls Natrium-Laurylsulfoacetat.
Ausgestaltung 4: Mischung nach Ausgestaltung 3, umfassend
   - 5 bis 30 Gew.-%, vorzugsweise 10 bis 20 Gew.-%, bevorzugt 12 bis 18 Gew.-%, besonders bevorzugt 13 bis 17 Gew.-% Pentan-1,2-diol,
   - 5 bis 30 Gew.-%, vorzugsweise 10 bis 20 Gew.-%, bevorzugt 12 bis 18 Gew.-%, besonders bevorzugt 13 bis 17 Gew.-% Natrium-Oleoylsarcosinat,
   - optional 3 bis 22 Gew.-%, vorzugsweise 5 bis 20 Gew.-%, bevorzugt 8 bis 20 Gew.-%, besonders bevorzugt 11 bis 17 Gew.-% Natrium-Laurylsulfoacetat,
   wobei sich die Gewichtsprozentangaben jeweils auf die Gesamtmasse der Mischung beziehen.
Ausgestaltung 5: Mischung nach Ausgestaltung 3, dadurch gekennzeichnet, dass das Gewichtsverhältnis von Pentan-1,2-diol zu Natrium-Oleoylsarcosinat im Bereich von 5 : 1 bis 1 : 5 liegt, vorzugsweise im Bereich von 3 : 1 bis 1 : 3, bevorzugt im Bereich von 2 : 1 bis 1 : 2, weiter bevorzugt im Bereich von 3 : 2 bis 2 : 3, besonders bevorzugt im Bereich von 4 : 3 bis 3 : 4, ganz besonders bevorzugt im Bereich von 8 : 7 bis 7 : 8.
Ausgestaltung 6: Mischung nach Ausgestaltung 3 oder 4, umfassend oder bestehend aus
   - 10 bis 20 Gew.-%, vorzugsweise 12 bis 18 Gew.-%, bevorzugt 13 bis 17 Gew.-%, besonders bevorzugt 14 bis 16 Gew.-% Pentan-1,2-diol,
   - 10 bis 20 Gew.-%, vorzugsweise 12 bis 18 Gew.-%, bevorzugt 13 bis 17 Gew.-%, besonders bevorzugt 13 bis 16 Gew.-% Natrium-Oleoylsarcosinat,
   - 5 bis 20 Gew.-%, vorzugsweise 8 bis 18 Gew.-%, bevorzugt 11 bis 17 Gew.-%, besonders bevorzugt 12 bis 16,5 Gew.-% Natrium-Laurylsulfoacetat,
   - 25 Gew.-% oder mehr Wasser, vorzugsweise 30 bis 60 Gew.-% Wasser, bevorzugt 35 bis 55 Gew.-% Wasser,
   - optional Natrium-Oleat, vorzugsweise 0,5 bis 5 Gew.-% Natrium-Oleat, bevorzugt 1 bis 3 Gew.-%,
   - optional Natriumchlorid, vorzugsweise 1 bis 5 Gew.-% Natriumchlorid, bevorzugt 1,5 bis 4 Gew.-%,
   - optional Dinatriumsulfoacetat, vorzugsweise 0,5 bis 3 Gew.-%, bevorzugt 0,6 bis 2 Gew.-%,
   - optional Natriumsulfat, vorzugsweise 0,5 bis 3 Gew.-%, bevorzugt 1 bis 2 Gew.-%,
   wobei sich die Gewichtsprozentangaben jeweils auf die Gesamtmasse der Mischung beziehen.
Ausgestaltung 7: Mischung nach einer der Ausgestaltungen 3 bis 6, dadurch gekennzeichnet, dass die Mischung einen pH-Wert von 6 oder höher hat, vorzugsweise einen pH-Wert von 6,5 oder höher.
Ausgestaltung 8: Zusammensetzung zur Herstellung einer kosmetischen Zubereitung, vorzugsweise eines Körperpflegemittels, umfassend eine Mischung nach einer der Ausgestaltungen 3 bis 7 sowie eine oder mehrere organische Substanzen mit einem log K_{OW} - Wert im Bereich von 1 bis 12, vorzugsweise im Bereich von 2 bis 10, besonders bevorzugt im Bereich von 3 bis 8.
Ausgestaltung 9: Zusammensetzung nach Ausgestaltung 8, dadurch gekennzeichnet, die Zusammensetzung transparent ist, vorzugsweise eine Trübung von weniger als 10 NTU (nephelometric turbidity units) aufweist, bevorzugt von weniger als 5 NTU, besonders bevorzugt von weniger als 3 NTU.
Ausgestaltung 10: Zusammensetzung nach Ausgestaltung 8 oder 9, dadurch gekennzeichnet, dass die Gesamtmenge der organischen Substanz(en) mit einem log K_{OW} - Wert im Bereich von 1 bis 12 in einem Bereich von 0,1 bis 5 Gew.-%, vorzugsweise in einem Bereich von 0,1 bis 3 Gew.-%, bevorzugt in einem Bereich von 0,15 bis 1 Gew.-%, besonders bevorzugt in einem Bereich von 0,2 bis 0,8 Gew.-%, jeweils bezogen auf die Gesamtmasse der Zusammensetzung liegt.
Ausgestaltung 11: Kosmetische Zubereitung, umfassend
   (i) eine Mischung nach einer der Ausgestaltungen 3 bis 7 sowie eine oder mehrere organische Substanzen mit einem log K_{OW} - Wert im Bereich von 1 bis 12, vorzugsweise im Bereich von 2 bis 10, besonders bevorzugt im Bereich von 3 bis 8
      oder
      eine Zusammensetzung nach einer der Ausgestaltungen 8 bis 10
      sowie
   (ii) gegebenenfalls weitere kosmetisch wirksame Bestandteile,
   (iii) gegebenenfalls sonstige Bestandteile.
Ausgestaltung 12: Kosmetische Zubereitung nach Ausgestaltung 11, dadurch gekennzeichnet, dass die kosmetische Zubereitung ausgewählt ist aus der Gruppe der Körperpflegemittel, vorzugsweise ausgewählt ist aus der Gruppe bestehend aus wässrigen und wässrig-alkoholischen Systemen wie Duschgele, Shampoos, alkoholische Parfüms, wässrig alkoholische Parfüms, After Shave Gele, Cremes, Lotionen, Haarpflegemittel, Haarstylingmittel.
Ausgestaltung 13: Kosmetische Zubereitung nach Ausgestaltung 11 oder 12, dadurch gekennzeichnet, dass die kosmetische Zubereitung, vorzugsweise das Körperpflegemittel,
   0,1 bis 15 Gew.-%, bevorzugt 0,5 bis 7 Gew.-%, weiter bevorzugt 0,5 bis 5 Gew.-%, und besonders bevorzugt 1 bis 3 Gew.-% einer Mischung nach einer der Ausgestaltungen 3 bis 7
   oder
   0,1 bis 15 Gew.-.%, bevorzugt 0,5 bis 7 Gew.-%, weiter bevorzugt 0,5 bis 5 Gew.-%, und besonders bevorzugt 1 bis 3 Gew.-% einer Zusammensetzung nach einer der Ausgestaltungen 8 bis 10
   umfasst, wobei sich die Gewichtsprozentangaben jeweils auf die Gesamtmasse der kosmetischen Zubereitung beziehen.
Ausgestaltung 14: Verfahren zur Erhöhung der Löslichkeit einer organischen Substanz und/oder zur Verringerung der Trübung einer Zubereitung, enthaltend eine organische Substanz und/oder zur Erhöhung der Transparenz einer Zubereitung enthaltend eine oder mehrere organische Substanzen,
   wobei die organische Substanz jeweils einen log K_{OW} - Wert im Bereich von 1 bis 12 aufweist, vorzugsweise im Bereich von 2 bis 10, besonders bevorzugt im Bereich von 3 bis 8,
   umfassend den Schritt:
   - Vermischen der organischen Substanz(en) mit einer Mischung nach einer der Ausgestaltungen 3 bis 7.
Ausgestaltung 15: Verfahren zum Herstellen einer Mischung gemäß einer der Ausgestaltungen 3 bis 7, einer Zusammensetzung gemäß einer der Ausgestaltungen 8 bis 10 oder einer kosmetischen Zubereitung gemäß einer der Ausgestaltungen 11 bis 13, umfassend den Schritt:
   - Mischen von Pentan-1,2-diol und Natrium-Oleoylsarcosinat sowie gegebenenfalls Natrium-Laurylsulfoacetat.
Ausgestaltung 16: Verwendung von Pentan-1,2-diol zur Verbesserung der lösungsvermittelnden Wirkung, insbesondere der Lösefähigkeit, von Tensiden, vorzugsweise von Natrium-Oleoylsarcosinat, Natrium-Laurylsulfoacetat oder deren Mischung, bevorzugt einer Mischung von Natrium-Oleoylsarcosinat und Natrium-Laurylsulfoacetat im Gewichtsverhältnis von 10 : 1 bis 1 : 4, bevorzugt im Gewichtsverhältnis von 4 : 1 bis 1 : 2.

Die Erfindung wird nachfolgend anhand von Beispielen näher erläutert.

### Beispiele

Sofern nicht anders angegeben, beziehen sich alle Angaben auf das Gewicht.

Es nachfolgend eingesetzten Rohstoffe sind kommerziell verfügbar.

Das im Folgenden eingesetzte "Oleoylsarcosinat HH" bestand aus 54 Gew.-% Natrium-Oleoylsarcosinat, 6 Gew.-% Natrium-Oleat und 40 Gew.-% Wasser.

Das im Folgenden eingesetzte "Laurylsulfoacetat VV" enthielt etwa 67 Gew.-% an Natrium-Laurylsulfoacetat, etwa 14 Gew.-% Natriumchlorid, 3-10 Gew.-% an Dinatriumsulfoacetat sowie 7 Gew.-% an Natriumsulfat und wies einen Wassergehalt von weniger als 1,5 Gew.-% auf.

### Beispiel 1: Lösungsvermittler (erfindungsgemäß und Vergleichsversuche)

| **Rohstoff** | **INCI** | **U.53787M Gew.-%** | **Z1 Gew.-%** | **Z2 Gew.-%** |
|---|---|---|---|---|
| Oleoylsarcosinat HH | Sodium Oleoyl Sarcosinate, Sodium Oleate | 25,00 | 25,00 | 25,00 |
| Hydrolite 5^{®} (Pentan-1,2-diol) | Pentylene Glycol | 15,00 | 15,00 | 15,00 |
| 1,2-Propylenglcol | Propylene Glycol | - | 15,00 | - |
| Glycerin | Glycerin | - | - | 15,00 |
| Laurylsulfoacetat VV | Sodium Lauryl Sulfoacetate, Sodium Chloride, Disodium Sulfoacetate, Sodium Sulfate | 20,00 | 20,00 | 20,00 |
| Demineralisiertes Wasser | Aqua | Ad 100 | Ad 100 | Ad 100 |

Die erfindungsgemäße Mischung U.53787M (pH-Wert: 5,5) war hellgelb und klar (transparent), wohingegen die nicht erfindungsgemäßen Mischungen Z1 und Z2 trüb waren und nicht vollständig gelöste Feststoffe-Bestandteile enthielten. Die erfindungsgemäße Mischung U.53787M blieb über einen Lagerungszeitraum von 6 Monaten bei Tageslicht und 5°C, 25°C bzw. 40°C klar und stabil.

Beispiel 2: Erfindungsgemäße Mischungen (Lösungsvermittler)

| **Rohstoff** | **INCI** | **A Gew.-%** | **B Gew.-%** | **C Gew.-%** |
|---|---|---|---|---|
| Oleoylsarcosinat HH | Sodium Oleoyl Sarcosinate, Sodium Oleate | 25,00 | 25,00 | 25,00 |
| Hydrolite 5^{®} (Pentan-1,2-diol) | Pentylene Glycol | 15,00 | 15,00 | 15,00 |
| Laurylsulfoacetat VV | Sodium Lauryl Sulfoacetate, Sodium Chloride, Disodium Sulfoacetate, Sodium Sulfate | 20,00 | 20,00 | 20,00 |
| NaOH 50%ig in Wasser | Sodium Hydroxide | 1,11 | 1,55 | 2,00 |
| Demineralisiertes Wasser | Aqua | Ad 100 | Ad 100 | Ad 100 |
| pH-Wert | | 6,0 | 6,5 | 7,5 |

Diese erfindungsgemäßen Mischungen waren klar (transparent) und blieben über einen Lagerungszeitraum von 6 Monaten bei Tageslicht klar und stabil. Die erfindungsgemäßen Mischungen A, B und C zeigten ein weiter verbessertes Lösungsvermögen und noch bessere lösungsvermittelnde Eigenschaften als U.53787M aus Beispiel 1.

### Beispiel 3: Lagerstabilität erfindungsgemäßer Zusammensetzungen und Zubereitungen

Die Transparenz (Klarheit) der Zusammensetzungen wurde nach 3 Monaten bei der jeweils angegebenen konstanten Lagerungstemperatur ermittelt.

### Beispiel 3.1: Untersuchungen mit Menthyllactat

| | **Gew.-%** | **Gew.-%** | **Gew.-%** |
|---|---|---|---|
| Frescolat^{®} ML (Menthyllactat) | 0,50 | 0,40 | 0,40 |
| Lösungsvermittler U.53787M aus Beispiel 1 | - | 2,00 | 2,00 |
| Lösungsvermittler B aus Beispiel 2 | 2,00 | - | - |
| Glycerin | 5,00 | 5,00 | - |
| Pentan-1,2-diol | - | - | 5,00 |
| 1,2-Propylenglycol | - | - | - |
| Wasser | Ad 100 | Ad 100 | Ad 100 |

| **Bewertung der Trübung** | | | |
|---|---|---|---|
| Herstellung bei 25° | klar | klar | klar |
| Lagerung bei 5°C | klar | klar | klar |
| Lagerung bei 40°C | klar | klar | klar |

### Beispiel 3.2: Untersuchungen mit Dragocid^{™} Liquid

| | **Gew.-%** | **Gew.-%** | **Gew.-%** | **Gew.-%** |
|---|---|---|---|---|
| Dragocid^{™} Liquid | 1,20 | 1,20 | 1,20 | 1,20 |
| Lösungsvermittler U.53787M aus Beispiel 1 | 2,00 | 2,00 | 2,00 | 2,00 |
| Glycerin | 5,00 | - | - | - |
| Pentan-1,2-diol | - | 0,50 | 3,00 | - |
| 1,2-Propylenglycol | - | - | - | 0,50 |
| Wasser | Ad 100 | Ad 100 | Ad 100 | Ad 100 |

| **Bewertung der Trübung** | | | | |
|---|---|---|---|---|
| Herstellung bei 25° | klar | klar | klar | klar |
| Lagerung bei 5°C | klar | klar | klar | klar |
| Lagerung bei 40°C | klar | klar | klar | klar |

Dragocid^{™} Liquid ist ein von Symrise kommerziell erhältliches Gemisch aus Phenoxyethanol, Methylparaben, Ethylparaben, Butylparaben, Propylparaben und Isobutylparaben.

### Beispiel 3.3: Untersuchungen mit Bisabolol

| | **Gew.-%** | **Gew.-%** | **Gew.-%** | **Gew.-%** |
|---|---|---|---|---|
| Bisabolol | 0,10 | 0,10 | 0,10 | 0,10 |
| Lösungsvermittler U.53787M aus Beispiel 1 | 2,00 | 2,00 | 2,00 | 2,00 |
| Pentan-1,2-diol | 0,50 | 2,50 | - | - |
| 1,2-Propylenglycol | - | - | 3,00 | 1,00 |
| Wasser | Ad 100 | Ad 100 | Ad 100 | Ad 100 |

| **Bewertung der Trübung** | | | | |
|---|---|---|---|---|
| Herstellung bei 25° | klar | klar | klar | klar |
| Lagerung bei 5°C | klar | klar | klar | klar |
| Lagerung bei 40°C | klar | klar | klar | klar |

| | **Gew.-%** | **Gew.-%** |
|---|---|---|
| Bisabolol | 0,10 | 0,20 |
| Lösungsvermittler U.53787M aus Beispiel 1 | 2,00 | - |
| Lösungsvermittler A aus Beispiel 2 | - | 2,00 |
| Glycerin | 4,00 | 5,00 |
| Wasser | Ad 100 | Ad 100 |

| **Bewertung der Trübung** | | |
|---|---|---|
| Herstellung bei 25° | klar | klar |
| Lagerung bei 5°C | klar | klar |
| Lagerung bei 40°C | klar | klar |

### Beispiel 3.4: Untersuchungen mit weiteren organischen Substanzen

| | **Gew.-%** |
|---|---|
| Zu untersuchende organische Substanz | "X,XX" |
| Lösungsvermittler U.53787M aus Beispiel 1 | 2,00 |
| Glycerin | 5,00 |
| Wasser | Ad 100 |

| **Bewertung der Trübung** | |
|---|---|
| Herstellung bei 25° | klar |
| Lagerung bei 5°C | klar |
| Lagerung bei 40°C | klar |

In diese Rahmenformulierung wurden die folgenden zu untersuchenden organischen Substanzen, jeweils in einer Menge von "x,xx Gew.-%" eingearbeitet, wobei die jeweilige Zusammensetzung bzw. Zubereitung bei der jeweils angegebenen konstanten Temperatur über den Lagerungszeitraum klar und stabil blieb:
Salbeiöl (Salvia Officinalis Oil) (0,10 Gew.-%), Rosenöl (Rosa Damascena Flower Oil) (0,20 Gew.-%), Parfümöl Lait de Rose (0,20 Gew.-%), Parfümöl Lait Vitamine Sun (0,30 Gew.-%), Parfümöl L'Eau d'été (0,20 Gew.-%), Parfümöl Jasmin Flowers (0,30 Gew.-%), Paraffinöl (Paraffinum Liquidum) (0,10 Gew.-%) bzw. Crinipan^{®} AD (Schuppenwirkstoff Climbazol) (0,10 Gew.-%).

### Formulierungsbeispiele:

In den nachfolgenden Formulierungsbeispielen war die jeweilige Phase enthaltend den erfindungsgemäßen PEG-freien Lösungsvermittler U.53787M aus Beispiel 1 klar und wiesen einen Trübungswert von kleiner als 3 NTU auf (wie oben definiert).

Die fertigen Formulierungen der Formulierungsbeispiele F1 bis F9 wiesen einen Trübungswert von kleiner als 10 NTU (wie oben definiert) (Beispiele F1, F2, F5, F7 und F9) bzw. von kleiner als 3 NTU (Formulierungsbeispiele F3, F4, F6 und F8) auf. Lediglich die weiss-gelbe Emulsion des Formulierungsbeispiels F10 wies einen Trübungswert von >10 NTU auf.

### Formulierungsbeispiel F1: After Shave Gel

| **Phase** | **Rohstoff** | **INCI** | **Gew.-%** |
|---|---|---|---|
| **A** | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium Sulfate | 7,00 |
| | Hydrolite® 5 | Pentylene Glycol | 5,00 |
| | SymSitive® | Pentylene Glycol, 4-t-Butylcyclohexanol | 1,00 |
| | Vitamin-E-Acetat | Tocopheryl Acetate | 0,50 |
| | Frescolat® ML | Menthyl Lactate | 0,50 |
| | Parfüm | Fragrance | 0,15 |
| | Glycerin | Glycerin | 5,00 |
| **B** | Wasser | Water (Aqua) | Ad 100 |
| | Pemulen TR2 | Acrylates/C10-30 Alkyl Acrylate Crosspolymer | 1,00 |
| | Extrapone® Glacier Water GW | Glycerin, Aqua | 1,00 |
| | SymCalmin® | Butylene Glycol, Pentylene Glycol, Hydroxyphenyl Propamidobenzoic Acid | 0,50 |
| | NaOH 10%ig in Wasser | Sodium Hydroxide | 3,15 |
| **C** | Tegosoft PC41 | Polyglyceryl-4 Caprate | 2,00 |
| **D** | Ethanol | Alcohol | 4,80 |
| | Blauer Farbstoff 1%ig in Wasser | Colour | 0,50 |

Herstellung: Inhaltsstoffe der Phase A unter Rühren klar lösen. Für Phase B Verdicker im Wasser quellen lassen, die restlichen Komponenten unter Rühren zugeben. Die Phase A zu Phase B unter Rühren zugeben, Phase C und Phase D nacheinander unter Rühren zudosieren.

Der pH-Wert des Formulierungsbeispiels F1 lag bei 5,9.

### Formulierungsbeispiel F2: Sonnenschutz-Gel mit Lichtschutzfaktor SPF 20-25

| **Phase** | **Rohstoff** | **INCI** | **Gew.-%** |
|---|---|---|---|
| **A** | Wasser | Water (Aqua) | Ad 100 |
| | Neo Heliopan® AP, 10% in Wasser, neutralisiert mit NaOH | Disodium Phenyl DibenzimidazoleTetrasulfonate | 30,00 |
| | Neo Heliopan® Hydro, 15% in Wasser, neutralisiert mit NaOH | Phenylbenzimidazole Sulfonic Acid | 33,34 |
| | EDTA BD | Disodium EDTA | 0,10 |
| | Dragosine | Carnosine | 0,20 |
| **B** | Esaflor HDR | Hydroxypropyl Guar | 1,50 |
| **C** | Biotive® Troxerutin | Troxerutin | 1,60 |
| | Roter Farbstoff | Colour | 0,10 |
| **D** | Hydrolite® 5 | Pentylene Glycol | 5,00 |
| | Parfüm | Fragrance | 0,10 |
| | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium Sulfate | 0,50 |
| | SymDiol® 68 | 1,2-Hexanediol, Caprylyl Glycol | 1,00 |

Herstellung: Inhaltsstoffe der Phase A zusammen geben. Den Verdicker aus Phase B unter Rühren zugeben und quellen lassen. Anschließend die Komponenten der Phase C nacheinander unter Rühren zudosieren. Für Phase D die Komponenten klar lösen und unter Rühren zu Phase A/B/C geben.

Der pH-Wert des Formulierungsbeispiels F2 lag bei 7,6.

### Formulierungsbeispiel F3: After Sun Gel

| **Phase** | **Rohstoff** | **INCI** | **Gew.-%** |
|---|---|---|---|
| **A** | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium | 15,00 |
| | | Oleate, Sodium Sulfate | |
| | *Glycerin | Glycerin | 5,00 |
| | Naürliches Alpha-Bisabolol | Bisabolol | 0,10 |
| | Vitamin-E-Acetat | Tocopheryl Acetate | 0,50 |
| | SymRepair® | Hexyldecanol, Bisabolol, Cetylhydroxyproline Palmitamide, Stearic Acid, Brassica Campestris Sterols | 0,50 |
| | SymHelios® 1031 | Benzylidene Dimethoxydimethylindanone | 0,10 |
| | Parfüm | Fragrance | 0,20 |
| **B** | Wasser | Water (Aqua) | Ad 100 |
| | Pemulen TR2 | Acrylates/C10-30 Alkyl Acrylate Crosspolymer | 1,00 |
| | D-Panthenol | Panthenol, Aqua | 0,50 |
| | Flowerpone® Frangipani | Aqua, Pentylene Glycol, PEG-40 Hydrogenated Castor Oil, Trideceth-9, Bisabolol, Plumeria Acutifolia Flower Extract | 1,00 |
| | Extrapone® Pearl GW | Aqua, Glycerin, Hydrolyzed Pearl, Xanthan Gum | 1,00 |
| | NaOH 10%ig in Wasser | Sodium Hydroxide | 3,00 |
| **C** | Ethanol | Alcohol | 14,40 |

Herstellung: Inhaltsstoffe der Phase A unter Rühren klar lösen. Für Phase B Verdicker im Wasser quellen lassen, die restlichen Komponenten unter Rühren zugeben. Die Phase A zu Phase B unter Rühren zugeben, Phase C unter Rühren zudosieren.

Der pH-Wert des Formulierungsbeispiels F3 lag bei 5,8.

### Formulierungsbeispiel F4: Klares Körpertonikum (Clear Body Tonic)

| **Phase** | **Rohstoff** | **INCI** | **Gew.-%** |
|---|---|---|---|
| **A** | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium Sulfate | 7,00 |
| | Frescolat® ML | Menthyl Lactate | 0,80 |
| | Parfüm | Fragrance | 1,00 |
| **B** | Wasser | Water (Aqua) | Ad 100 |
| | Hydrolite® 5 | Pentylene Glycol | 5,00 |
| | Extrapone® Bamboo | Propylene Glycol, Aqua, Butylene Glycol, Bambusa Vulgaris Shoot Extract | 1,00 |
| | Extrapone® Guarana | Aqua, Propylene Glycol, Paullinia Cupana Seed Extract, Alcohol | 1,00 |
| **C** | Ethanol | Alcohol | 25,00 |
| | Azorubin 0,1%ig | Colour | 0,10 |

Herstellung: Inhaltsstoffe der Phase A unter Rühren klar lösen. Die Komponenten der Phase B mischen und zu Phase A unter Rühren zugeben, Phase C nacheinander unter Rühren zudosieren.

Der pH-Wert des Formulierungsbeispiels F4 lag bei 6,0.

### Formulierungsbeispiel F5: Selbstbräunungsgel (Self Tan Gel)

| **Phase** | **Rohstoff** | **INCI** | **Gew.-%** |
|---|---|---|---|
| **A** | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium Sulfate | 15,00 |
| | Vitamin E Acetat | Tocopheryl Acetate | 0,50 |
| | Alpha-Bisabolol Natural | Bisabolol | 0,10 |
| | SymHelios® 1031 | Benzylidene Dimethoxydimethylindanone | 0,10 |
| | Hydrolite® 5 | Pentylene Glycol | 2,50 |
| | Glycerin | Glycerin | 5,00 |
| | Parfüm | Fragrance | 0,20 |
| **B** | Wasser | Water (Aqua) | Ad 100 |
| | Pemulen TR2 | Acrylates/C10-30 Alkyl Acrylate Crosspolymer | 1,00 |
| | Dihydroxyaceton | Dihydroxyacetone | 5,00 |
| | D-Panthenol | Panthenol | 0,50 |
| | Extrapone® Lotus Flower | | 1,00 |
| | NaOH 10%ig in Wasser | Sodium Hydroxide | 0,70 |
| **C** | Ethanol | Alcohol | 5,00 |

Herstellung: Inhaltsstoffe der Phase A unter Rühren klar lösen. Für Phase B Verdicker im Wasser quellen lassen, die restlichen Komponenten unter Rühren zugeben. Die Phase A zu Phase B unter Rühren zugeben, Phase C unter Rühren zudosieren.

Der pH-Wert des Formulierungsbeispiels F5 lag bei 5,0.

### Formulierungsbeispiel F6: Tränkungslösung für Baby-Pflege Tücher (Baby Wipes)

| **Phase** | **Rohstoff** | **INCI** | **Gew.-%** |
|---|---|---|---|
| **A** | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium Sulfate | 10,00 |
| | Natürliches Alpha-Bisabolol | Bisabolol | 0,10 |
| | Vitamin-E-Acetat | Tocopheryl Acetate | 0,50 |
| | Hydrolite® 5 | Pentylene Glycol | 5,00 |
| | Glycerin | Glycerin | 5,00 |
| | Tegosoft PC41 | Polyglyceryl-4 Caprate | 1,00 |
| **B** | Wasser | Water (Aqua) | Ad 100 |
| | D-Panthenol | Panthenol, Aqua | 0,80 |
| | DragoCalm® | Aqua, Glycerin, Avena Sativa Kernel Extract | 1,00 |
| | Allplant Essence®Org Rose Geranium P | Pelargonium Graveolens Flower/Leaf/Stem Water | 1,00 |

Herstellung: Inhaltsstoffe der Phase A unter Rühren klar lösen. Die Komponenten der Phase B mischen und zu Phase A unter Rühren zugeben.

Der pH-Wert des Formulierungsbeispiels F6 lag bei 6,2.

### Formulierungsbeispiel F7: Alkoholfreies Körpergel (Body Gel)

| **Phase** | **Rohstoff** | **INCI** | **Gew.-%** |
|---|---|---|---|
| **A** | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium Sulfate | 10,00 |
| | Orangenöl Guinea | Citrus Aurantium Dulcis | 0,25 |
| | Lavendelöl kroatisch | Lavandula Hybrida Oil | 0,10 |
| | Hydrolite® 5 | Pentylene Glycol | 5,00 |
| | Vitamin-E-Acetat | Tocopheryl Acetate | 0,50 |
| | Tegosoft PC41 | Polyglyceryl-4-Caprate | 2,00 |
| **B** | Wasser | Water (Aqua) | Ad 100 |
| | Keltrol CG T | Xanthan Gum | 0,50 |
| | Aristoflex TAC | Ammonium Acryloyl Dimethyltaurate/ Carboxyethyl Acrylate Crosspolymer | 1,50 |
| | Aloe Vera Gel Concentrate 10/1 | Aloe Barbadensis Gel | 0,50 |
| | Dragosine | Carnosine | 0,10 |
| | SymGlucan® | Aqua, Glycerin, Beta-Glucan | 2,00 |
| | D-Panthenol | Panthenol, Aqua | 0,50 |
| | Farbstoff "Orange No.4" 0,1%ig | Colour | 0,10 |

Herstellung: Inhaltsstoffe der Phase A unter Rühren klar lösen. Für Phase B Verdicker im Wasser quellen lassen, die restlichen Komponenten unter Rühren zugeben. Die Phase A zu Phase B unter Rühren zugeben.

Der pH-Wert des Formulierungsbeispiels F7 lag bei 6,0.

### Formulierungsbeispiel F8: Kosmetische Zubereitung mit Pflanzenextrakten

| **Phase** | **Rohstoff** | **INCI** | **Gew.-%** |
|---|---|---|---|
| **A** | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium Sulfate | 10,00 |
| | Schafgarbenöl | Achillea Millefolium Oil | 0,10 |
| | Kamillenöl ägyptisch | Chamomilla Recutita Flower Oil | 0,20 |
| | Natürliches Alpha-Bisabolol | Bisabolol | 0,10 |
| **B** | SymDiol® 68 | 1,2-Hexanediol, Caprylyl Glycol | 0,50 |
| | 1,2-Butylenglycol | Butylene Glycol | 40,00 |
| **C** | Wasser | Water (Aqua) | Ad 100 |

Herstellung: Inhaltsstoffe der Phase A unter Rühren klar lösen. Die Komponenten der Phase B mischen und zu Phase A unter Rühren zugeben. Die Phase C unter Rühren zudosieren.

Der pH-Wert des Formulierungsbeispiels F8 lag bei 6,2.

### Formulierungsbeispiel F9: Styling Gel

| **Phase** | **Rohstoff** | **INCI** | **Gew.-%** |
|---|---|---|---|
| **A** | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium Sulfate | 7,00 |
| | Hydrolite® 5 | Pentylene Glycol | 2,50 |
| | SymRepair® | Hexyldecanol, Bisabolol, Cetylhydroxyproline Palmitamide, Stearic Acid, Brassica Campestris Sterols | 1,00 |
| | Parfüm | Fragrance | 0,30 |
| **B** | Wasser | Water (Aqua) | Ad 100 |
| | Amaze XT | Dehydroxanthan Gum | 2,00 |
| | Hubridur 875 | Polyurethane-2 and Polymethyl Methacrylate | 5,00 |
| | Dragoderm® | Glycerin, Triticum Vulgare Gluten, Aqua | 2,00 |
| | Extrapone® Silk | Aqua, Glycerin, Hydrolyzed Silk | 1,00 |
| | Symdiol® 68 | 1,2-Hexanediol, Caprylyl Glycol | 1,00 |
| **C** | Ethanol | Alcohol | 5,00 |

Herstellung: Inhaltsstoffe der Phase A unter Rühren klar lösen. Für Phase B Verdicker im Wasser quellen lassen, die restlichen Komponenten unter Rühren zugeben. Die Phase A zu Phase B unter Rühren zugeben, Phase C unter Rühren zudosieren.

Der pH-Wert des Formulierungsbeispiels F9 lag bei 6,6.

### Formulierungsbeispiel F10: Sonnenschutzemulsion

| **Phase** | **Rohstoff** | **INCI** | **Gew.-%** |
|---|---|---|---|
| **A** | Emulsiphos | Potassium Cetyl Phosphate, Hydrogenated Palm Glycerides | 2,50 |
| | Cithrol GMM | Glyceryl Myristate | 1,00 |
| | Neo Heliopan® 357 | Butyl Methoxydibenzoylmethane | 5,00 |
| | Neo Heliopan® OS | Ethylhexyl Salicylate | 5,00 |
| | Neo Heliopan® 303 | Octocrylene | 10,00 |
| | Neo Heliopan® HMS | Homosalate | 10,00 |
| | Tinosorb® S | Bis-Ethylhexyloxyphenol Methoxyphenyl Triazine | 4,00 |
| | Neo Heliopan® E 1000 | Isoamyl p-Methoxycinnamate | 2,00 |
| | Dragoxat® 89 | Ethylhexyl Isononanoate | 3,00 |
| | SymMollient® S | Cetearyl Nonanoate | 2,00 |
| | Antaron WP 660 | Tricontanyl PVP | 2,00 |
| | Xiameter PMX-0246 | Cyclohexasiloxane (and) Cyclopentasiloxane | 2,00 |
| | KF-995 | Cyclopentasiloxane | 2,00 |
| | Silcare Silicone 41M65 | Stearyl Dimethicone | 1,00 |
| | EDETA BD | Disodium EDTA | 0,10 |
| | Vitamin E Acetat | Tocopheryl Acetate | 0,50 |
| | Asensa PR 200 | Polyethylene | 1,00 |
| | Keltrol T | Xanthan Gum | 0,50 |
| | Eusolex T AVO | Titanium Dioxide, Silica | 2,00 |
| **B** | Wasser | Aqua (Water) | 21,86 |
| | Neo Heliopan Hydro, 15% in Wasser, neutralisiert mit NaOH | Phenylbenzimidazole Sulfonic Acid | 13,34 |
| | Gycerin | Glycerin | 3,00 |
| | D-Panthenol 75L | Panthenol | 0,60 |
| | NaOH 10% in Wasser | Sodium Hydroxide | 0,90 |
| | PEG-freier Lösungsvermittler U.53787M aus Beispiel 1 | Aqua, Pentylene Glycol, Sodium Lauryl Sulfoacetate, Sodium Oleoyl Sarcosinate, Sodium Chloride, Disodium Sulfoacetate, Sodium Oleate, Sodium | 2,00 |
| | | Sulfate | |
| **C** | Phenoxyethanol | Phenoxyethanol | 0,75 |
| | SymClariol | Decylene Glycol | 0,25 |
| **D** | Parfüm | Fragrance | 0,20 |
| | Orgasol Caresse | Polyamide-5 | 1,50 |

Herstellung: Inhaltsstoffe der Phase A und der Phase B separat bei 80°C lösen und unter Rühren zusammengeben. Die Phase C bei 60°C zu Phase A/B unter Rühren zugeben und anschließend homogenisieren. Unter Rühren auf Raumtemperatur abkühlen lassen. Die Phase D zudosieren und nochmals homogenisieren.

Der pH-Wert des Formulierungsbeispiels F10 lag bei 7,3.

## Patentansprüche

1. Verwendung von (a) einem oder mehreren 1,2-Alkandiolen mit 5 bis 8 C-Atomen in Kombination mit (b) einer oder mehreren Verbindungen aus der Gruppe bestehend aus Natrium- oder Kalium-R^{alk}-sarcosinaten, wobei das Strukturelement R^{alk} Alkanoyl, Alkenoyl oder Alkadienoyl mit 12 bis 22 C-Atomen bedeutet, Natrium- oder Kalium-C10-C16-Alkyl-sulfoacetaten und deren Mischung
- als Lösungsvermittler für eine oder mehrere organische Substanzen,
- zur Erhöhung der Löslichkeit einer oder mehrerer organischer Substanzen,
- zur Verringerung der Trübung einer Zubereitung, vorzugsweise einer Dispersion und/oder Emulsion, enthaltend eine oder mehrere organische Substanzen,
und/oder
- zur Erhöhung der Transparenz einer Zubereitung, vorzugsweise einer Dispersion und/oder Emulsion, enthaltend eine oder mehrere organische Substanzen,
wobei die organischen Substanzen jeweils vorzugsweise einen log K_{OW} - Wert im Bereich von 1 bis 12 aufweisen, bevorzugt im Bereich von 2 bis 10, besonders bevorzugt im Bereich von 3 bis 8.

2. Verwendung nach Anspruch 1, **dadurch gekennzeichnet, dass** die eine oder mehrere organische Substanzen einen log K_{OW} - Wert im Bereich von 1 bis 12 aufweisen, vorzugsweise im Bereich von 2 bis 10, besonders bevorzugt im Bereich von 3 bis 8, und ausgewählt ist bzw. sind aus der Gruppe der zum Einsatz in kosmetischen Zubereitungen, vorzugsweise in Körperpflegemitteln, geeigneten Substanzen.

3. Mischung umfassend
(i) ein oder mehrere 1,2-Alkandiole mit 5 bis 8 C-Atomen,
(ii) ein oder mehrere Natrium- oder Kalium-R^{alk}-sarcosinate, wobei das Strukturelement R^{alk} Alkanoyl, Alkenoyl oder Alkadienoyl mit 12 bis 22 C-Atomen bedeutet, vorzugsweise mit 16 bis 22 C-Atomen, und
(iii) optional ein oder mehrere Natrium- oder Kalium-C10-C16-Alkyl-sulfoacetate.

4. Mischung nach Anspruch 3, umfassend
- 5 bis 30 Gew.-%, vorzugsweise 10 bis 20 Gew.-%, bevorzugt 12 bis 18 Gew.-%, besonders bevorzugt 13 bis 17 Gew.-% an Bestandteil (i), vorzugsweise Pentan-1,2-diol,
- 5 bis 30 Gew.-%, vorzugsweise 10 bis 20 Gew.-%, bevorzugt 12 bis 18 Gew.-%, besonders bevorzugt 13 bis 17 Gew.-% an Bestandteil (ii), vorzugsweise Natrium-Oleoylsarcosinat,
- optional 3 bis 22 Gew.-%, vorzugsweise 5 bis 20 Gew.-%, bevorzugt 8 bis 20 Gew.-%, besonders bevorzugt 11 bis 17 Gew.-% an Bestandteil (iii), vorzugsweise Natrium-Laurylsulfoacetat,
wobei sich die Gewichtsprozentangaben jeweils auf die Gesamtmasse der Mischung beziehen.

5. Mischung nach Anspruch 3, **dadurch gekennzeichnet, dass** das Gewichtsverhältnis von Bestandteil (i) zu Bestandteil (ii) im Bereich von 5 : 1 bis 1 : 5 liegt, vorzugsweise im Bereich von 3 : 1 bis 1 : 3, bevorzugt im Bereich von 2 : 1 bis 1 : 2, weiter bevorzugt im Bereich von 3 : 2 bis 2 : 3, besonders bevorzugt im Bereich von 4 : 3 bis 3 : 4, ganz besonders bevorzugt im Bereich von 8 : 7 bis 7 : 8.

6. Mischung nach Anspruch 3 oder 4, umfassend oder bestehend aus
- 10 bis 20 Gew.-%, vorzugsweise 12 bis 18 Gew.-%, bevorzugt 13 bis 17 Gew.-%, besonders bevorzugt 14 bis 16 Gew.-% an Bestandteil (i), vorzugsweise Pentan-1,2-diol,
- 10 bis 20 Gew.-%, vorzugsweise 12 bis 18 Gew.-%, bevorzugt 13 bis 17 Gew.-%, besonders bevorzugt 13 bis 16 Gew.-% an Bestandteil (ii), vorzugsweise Natrium-Oleoylsarcosinat,
- 5 bis 20 Gew.-%, vorzugsweise 8 bis 18 Gew.-%, bevorzugt 11 bis 17 Gew.-%, besonders bevorzugt 12 bis 16,5 Gew.-% an Bestandteil (iii), vorzugsweise Natrium-Laurylsulfoacetat,
- 25 Gew.-% oder mehr Wasser, vorzugsweise 30 bis 60 Gew.-% Wasser, bevorzugt 35 bis 55 Gew.-% Wasser,
- optional Natrium-Oleat, vorzugsweise 0,5 bis 5 Gew.-% Natrium-Oleat, bevorzugt 1 bis 3 Gew.-%,
- optional Natriumchlorid, vorzugsweise 1 bis 5 Gew.-% Natriumchlorid, bevorzugt 1,5 bis 4 Gew.-%,
- optional Dinatriumsulfoacetat, vorzugsweise 0,5 bis 3 Gew.-%, bevorzugt 0,6 bis 2 Gew.-%,
- optional Natriumsulfat, vorzugsweise 0,5 bis 3 Gew.-%, bevorzugt 1 bis 2 Gew.-%,
wobei sich die Gewichtsprozentangaben jeweils auf die Gesamtmasse der Mischung beziehen.

7. Mischung nach einem der Ansprüche 3 bis 6, **dadurch gekennzeichnet, dass** die Mischung einen pH-Wert von 6 oder höher hat, vorzugsweise einen pH-Wert von 6,5 oder höher.

8. Zusammensetzung zur Herstellung einer kosmetischen Zubereitung, vorzugsweise eines Körperpflegemittels, umfassend eine Mischung nach einem der Ansprüche 3 bis 7 sowie eine oder mehrere organische Substanzen mit einem log K_{OW} - Wert im Bereich von 1 bis 12, vorzugsweise im Bereich von 2 bis 10, besonders bevorzugt im Bereich von 3 bis 8.

9. Zusammensetzung nach Anspruch 8, **dadurch gekennzeichnet**, die Zusammensetzung transparent ist, vorzugsweise eine Trübung von weniger als 10 NTU (nephelometric turbidity units) aufweist, bevorzugt von weniger als 5 NTU, besonders bevorzugt von weniger als 3 NTU.

10. Zusammensetzung nach Anspruch 8 oder 9, **dadurch gekennzeichnet, dass** die Gesamtmenge der organischen Substanz(en) mit einem log K_{OW} - Wert im Bereich von 1 bis 12 in einem Bereich von 0,1 bis 5 Gew.-%, vorzugsweise in einem Bereich von 0,1 bis 3 Gew.-%, bevorzugt in einem Bereich von 0,15 bis 1 Gew.-%, besonders bevorzugt in einem Bereich von 0,2 bis 0,8 Gew.-%,
wobei sich die Gewichtsprozentangaben jeweils auf die Gesamtmasse der Zusammensetzung beziehen.

11. Kosmetische Zubereitung, umfassend
- eine Mischung nach einem der Ansprüche 3 bis 7 sowie eine oder mehrere organische Substanzen mit einem log K_{OW} - Wert im Bereich von 1 bis 12, vorzugsweise im Bereich von 2 bis 10, besonders bevorzugt im Bereich von 3 bis 8
oder
eine Zusammensetzung nach einem der Ansprüche 8 bis 10 sowie
gegebenenfalls weitere kosmetisch wirksame Bestandteile,
gegebenenfalls sonstige Bestandteile.

12. Kosmetische Zubereitung nach Anspruch 11, **dadurch gekennzeichnet, dass** die kosmetische Zubereitung ausgewählt ist aus der Gruppe der Körperpflegemittel, vorzugsweise ausgewählt ist aus der Gruppe bestehend aus wässrigen und wässrig-alkoholischen Systemen wie Duschgele, Shampoos, alkoholische Parfüms, wässrig alkoholische Parfüms, After Shave Gele, Cremes, Lotionen, Haarpflegemittel, Haarstylingmittel.

13. Kosmetische Zubereitung nach Anspruch 11 oder 12, **dadurch gekennzeichnet, dass** die Zubereitung, vorzugsweise das Körperpflegemittel, umfasst
0,1 bis 15 Gew.-%, bevorzugt 0,5 bis 7 Gew.-% und besonders bevorzugt 1 bis 3 Gew.-% einer Mischung nach einem der Ansprüche 3 bis 7,
oder
0,1 bis 15 Gew.-.%, bevorzugt 0,5 bis 7 Gew.-% und besonders bevorzugt 1 bis 3 Gew.-% einer Zusammensetzung nach einem der Ansprüche 8 bis 10,
wobei sich die Gewichtsprozentangaben jeweils auf die Gesamtmasse der kosmetischen Zubereitung beziehen.

14. Verfahren zur Erhöhung der Löslichkeit einer organischen Substanz und/oder zur Verringerung der Trübung einer Zubereitung, enthaltend eine organische Substanz und/oder zur Erhöhung der Transparenz einer Zubereitung enthaltend eine oder mehrere organische Substanzen,
wobei die organische Substanz jeweils einen log K_{OW} - Wert im Bereich von 1 bis 12 aufweist, vorzugsweise im Bereich von 2 bis 10, besonders bevorzugt im Bereich von 3 bis 8,
umfassend den Schritt:
- Vermischen der organischen Substanz(en) mit einer Mischung nach einem der Ansprüche 3 bis 7.

15. Verfahren zum Herstellen einer Mischung gemäß einem der Ansprüche 3 bis 7, einer Zusammensetzung gemäß einem der Ansprüche 8 bis 10 oder einer kosmetischen Zubereitung gemäß einem der Ansprüche 11 bis 13, umfassend den Schritt:
- Mischen von Bestandteil (i) und Bestandteil (ii) sowie gegebenenfalls Bestandteil (iii).

16. Verwendung eines 1,2-Alkandiols mit 5 bis 8 C-Atomen oder einer Mischung von 1,2-Alkandiolen mit 5 bis 8 C-Atomen zur Verbesserung der lösungsvermittelnden Wirkung von Tensiden, insbesondere der Lösefähigkeit von Tensiden, vorzugsweise von Natrium-Oleoylsarcosinat, Natrium-Laurylsulfoacetat oder deren Mischung, bevorzugt einer Mischung von Natrium-Oleoylsarcosinat und Natrium-Laurylsulfoacetat im Gewichtsverhältnis von 10 : 1 bis 1 : 4, bevorzugt im Gewichtsverhältnis von 4 : 1 bis 1 : 2.
